# EUROPEAN PATENT SPECIFICATION

(11) **EP 2 992 888 B1**
(45) Date of publication and mention of the grant of the patent: **01.02.2017**
(21) Application number: 14866552.4
(22) Date of filing: 16.04.2014
(51) Int. Cl.: A61K 31/704, A61P 3/10

(54) **USE OF PENTACYCLIC TRITERPENOID SAPONIN COMPOUND FROM SZECHUAN MELANDIUM ROOT FOR PREPARING HYPOGLYCEMIC DRUG**
VERWENDUNG EINER PENTACYCLISCHEN TRITERPENOIDSAPONINVERBINDUNG AUS DER SZECHUAN MELANDIUM-WURZEL ZUR HERSTELLUNG EINES BLUTZUCKERSENKENDEN ARZNEIMITTELS
UTILISATION D'UN COMPOSÉ SAPONINE TRITERPÉNOÏDE PENTACYCLIQUE DE RACINE DE SZECHUAN MELANDIUM POUR LA PRÉPARATION D'UN HYPOGLYCÉMIANT

(30) Priority: 28.11.2013 CN 201310627408
(43) Date of publication of application: 09.03.2016
(73) Proprietor: Wang, Xueyong, Beijing 100029 (CN); Zhao, Baosheng, Beijing 100029 (CN)
(72) Inventor: Wang, Xueyong, Beijing 100029 (CN); Zhao, Baosheng, Beijing 100029 (CN)
(74) Representative: Sun, Yiming
(86) International application number: PCT/CN2014/075471
(87) International publication number: WO 2015/078133

(56) References cited:
- CN-A- 101 817 862
- CN-A- 103 599 117
- WEI XU ET AL: "Pentacyclic Triterpenoid Saponins from Silene viscidula", HELVETICA CHIMICA ACTA, vol. 93, no. 10, 1 October 2010 (2010-10-01), pages 2007-2014, XP055248134, CH ISSN: 0018-019X, DOI: 10.1002/hlca.201000016
- J. ZHAO; NORIO NAKAMURA; MASAO HATTORI.: "New triterpenoid saponins from the roots of sinocrassula asclepiadea", PHARMACEUTICAL SOCIETY OF JAPAN, vol. 52, no. 2, 2004, pages 230-237, XP055248075,
- TONG WANG: "Study on the Chemical Constituents of Silene Szechuensis", MODERN CHINESE MEDICINE, vol. 11, no. 1, 1 January 2009 (2009-01-01), XP055239464,
- WANG, TONG ET AL.: 'Study on the Chemical Constituents of Silene Szechuensis (I' MODERN CHINESE MEDICINE vol. 11, no. 1, 31 January 2009, pages 9 - 12, XP055239464

## Description

### Field of the Invention

This invention relates to *Wacao* saponins and their compositions for use as hypoglycemic agent. The invention involves an anti-diabetic agent, an anti-diabetic composition containing the anti-diabetic agent, and said agents for use in the treatment of diabetes in particular application.

### Background

Diabetes mellitus is a glucose metabolic disorder characterized by abnormally high blood glucose level (hyperglycemia) in the fasting state or during an oral glucose tolerance tests (OGTT). Generally speaking, diabetes has two major types: type 1 diabetes mellitus (T1DM) and type 2 diabetes mellitus (T2DM). T1DM results from the decreased insulin and even the failure to produce insulin, a hormone which is responsible for metabolism and utilization of glucose, and whose deficiency inevitably leads to hyperglycemia. T2DM results from insulin resistance (IR), which usually, as a result, shows hyperinsulinemia, the high level of insulin in blood. IR means the main insulin sensitive tissues including liver, muscle and adipose tissues produce resistance to insulin stimulation in glucose and lipid metabolism. The consequence of IR is that the body has to secrete more insulin to compensate for IR; nevertheless, blood glucose level still increases abnormally.

At present, there are several anti-diabetic drugs to choose besides insulin. Insulinotropic sulfonylurea is widely used among the chemical drugs, which has the effect on anti-diabetes via stimulating the pancreatic β-cells to secrete more insulin to increase serum insulin levels, but it has the risk of causing patients hypoglycemia. Another widely used hypoglycemic agent is biguanides, such as metformin and phenformin. Its advantages are promoting the body's peripheral glucose utilization and amending the body's high blood glucose level, but will not increase the risk of hypoglycemia. It can be used in combination with insulin or insulin secretagogues, and the side effects of which are causing lactic acidosis, diarrhea and nausea. The other relatively new class of antidiabetic drugs are insulin sensitizer glitazones (thiazolidinediones, TZDs) such as rosiglitazone and pioglitazone. They can increase the sensitivity of tissues to insulin, and reduce the levels of fasting blood glucose and insulin and the postprandial by enhancing cells' glucose utilization, which, however, also have some adverse reactions including causing sodium retention, increasing blood volume, and adding cardiac load etc..

Plant-derived compound screening is an important way to find new antidiabetic drugs. Among them, saponins are worthy of attention. They can prevent and treat the diabetes by regulating blood lipids, improving IR, lowering blood glucose level and so on. Current studies show that Panax saponins, prunella triterpenoid saponins, ginseng saponins, oleanolic saponins, bitter saponins, and aralia saponins have a good hypoglycemic effect and can be developed into new antidiabetic drugs.

The patent application CN 101 817 862 A (2010, Univ China Pahrma) discloses the use of an ursolic acid-3-O-beta-D-pyranglucuronide and esters thereof for the treatment of diabetes. These compounds are reported to reduce hyperglycemia after meals.

*Silene viscidula* (Called *Wacao* in Chinese) is a species of plant belongs to the family Caryophyllaceae, the root of which is known as '*Wacao*' by the hmong people in China. *Wacao* has the effects of analgesia, hemostasia, clearing heat and diuretic, and it is often used to treat traumatic injury, rheumatic pain, bronchitis, and urinary tract infection in clinical application. Currently, researches of *Silene viscidula are* mainly concentrated on its chemical compositions, but barely in the pharmacological. The main chemical compositions of *Silene viscidula* are saponins, proteins, organic acids, polysaccharides, and cyclic peptides etc. Our former work had successfully isolated the saponins of sinocrassuloside VI, sinocrassuloside VII, sinocrassuloside VIII, sinocrassuloside IX, sinocrassuloside XII, and sinocrassuloside XIII. The cyclic peptide composition in *Silene viscidula* mainly comprises cyclic peptides A, B, C (silenins A, B, C). The steroidal ketones components in *Silene viscidula* mainly include 20- hydroxyecdysone, 1-epi-integristeroneA, abutasterone, stachysterone A, 15- hydroxystachysterone A; The organic acid constituents in *Silene viscidula* often include hydroxycinnamic acid, oleanolic acid and vanilla acid.

### Summary of the Invention

What the invention is intended to solve on the techniques is to provide the application of pentacyclic triterpenoid saponins extracted from *Silene viscidula* and the compounds as herein defined for manufacturing medicaments possessed anti-diabetic actives. Through mass screening and research, we had firstly discovered that the compounds of pentacyclic triterpenoid saponins, especially the compound based on the nucleus structure of sinocrassuloside and its analogues, extracted and purified from a plant of *Silence viscidula* have a strong hypoglycemic effect. Such compounds as herein defined and their compositions can be used to prepare the anti-diabetic drugs.

The analogues of compounds comprised of the mother nucleus structure of sinocrassulosides mainly refer to the sinocrassuloside modifications and its derivatives that mainly result from aglycone of sinocrassuloside binding different numbers and combinations of glucose. These compounds include sinocrassuloside VI, sinocrassuloside VII, sinocrassuloside VIII, sinocrassuloside IX, sinocrassuloside X, sinocrassuloside XI, sinocrassuloside XII, sinocrassuloside XIII and their said pharmaceutically acceptable salts.

Among them, compounds of sinocrassuloside VI and sinocrassuloside VII, sinocrassuloside VIII and sinocrassuloside IX, sinocrassuloside XII and sinocrassuloside XIII are three pairs of cis and trans isomers, all of which have good anti-diabetic activities and exhibit a certain quantitative structure-activity relationship.

The said 'pharmaceutically acceptable salt' in the present invention means compounds of the sinocrassuloside forming salts by alkali or alkaline earth metal. The alkaline includes sodium hydroxide, potassium hydroxide, calcium hydroxide, magnesium hydroxide, sodium bicarbonate, ammonium chloride or ammonia; and the alkaline earth metals include sodium, potassium, calcium, aluminum, copper, zinc or magnesium.

The protected pentacyclic triterpenoid compounds of *Wacao* saponins in the present invention mainly refer to the compounds which are extracted and purified from a plant of *Silene viscidula.* In addition, compounds of *Wacao* saponins extracted from other plants, obtained by chemical synthesis, semi-synthetic or biological transformation are also within the scope of the present invention.

The said *Wacao* saponin compounds are mainly suitable for the treatment of T2DM. The dose ranges from 0.1 mg/kg to 10mg/kg body weight.

The present invention discloses the use of *Wacao* saponin herein defined in the manufacture of hypoglycemic medicaments. The said *Wacao* saponins comprise as an active ingredient compound of general formula (A):

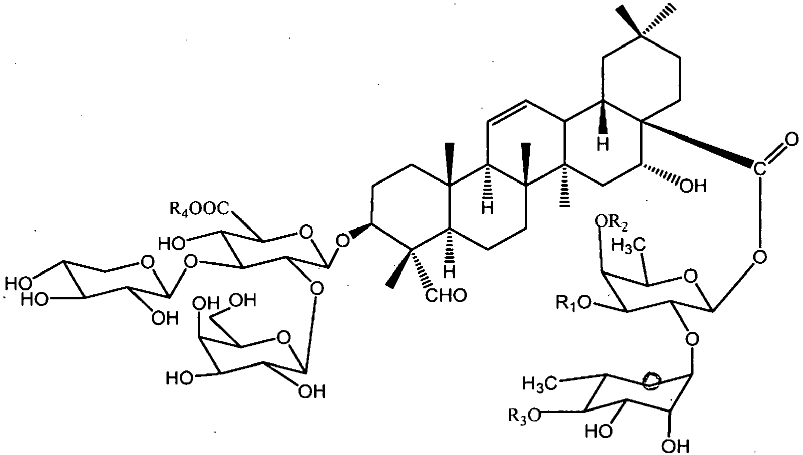

In the general formula (A):
R₁= H, Ac, Glc or any other organic group;
R₂= (E) -MC, (Z)-MC, or Ac;
R₃=H, or Xyl :
R₄=H, CH₃, or CH₂CH₂CH₂CH₃:

The AC herein defined refers to a group as follows:

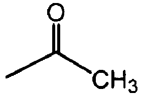

The (E) -MC herein defined refers to a group as follows:

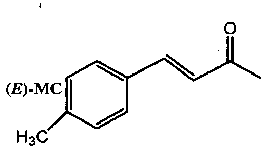

The (Z) -MC herein defined refers to a group as follows:

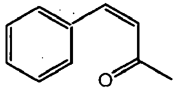

According to a further embodiment, the invention concerns as follows:

According to a further aspect, the said compound of *Wacao* saponin is represented by formula (1), hereinafter referred to as 'Compound (1)', the sinocrassuloside VI:

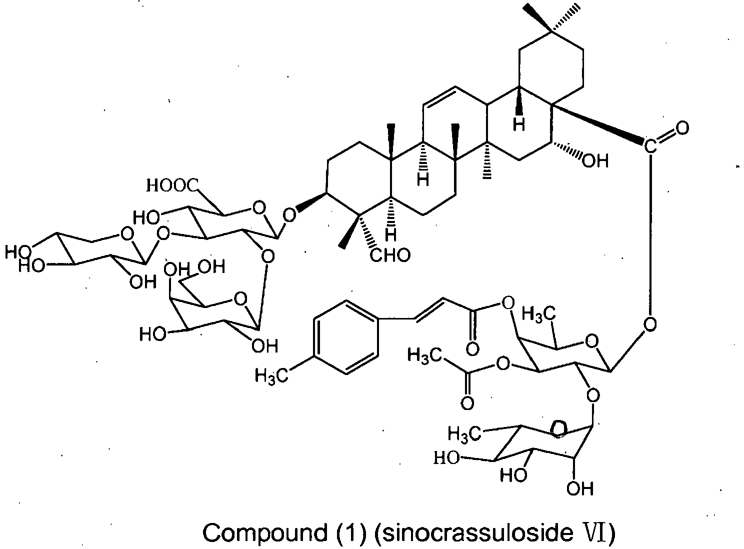

According to a further aspect, the said compound of *Wacao* saponins is represented by formula (2), hereinafter referred to as 'Compound (2)', the sinocrassuloside VII:

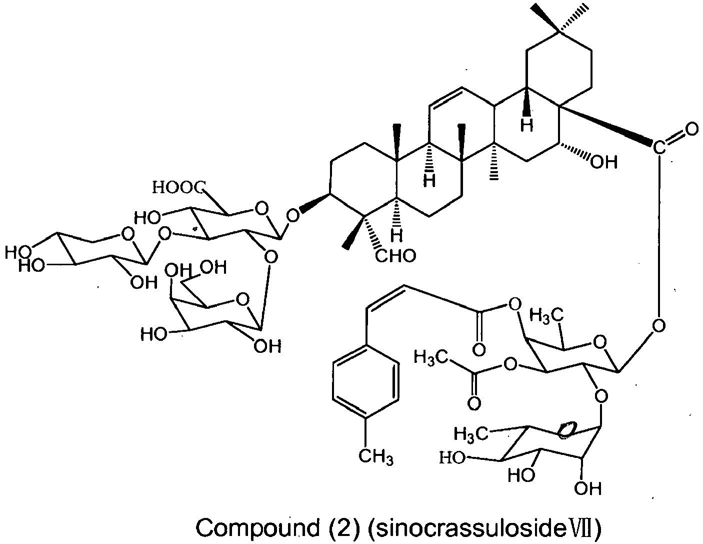

According to a further aspect, the said compound of *Wacao* saponin is represented by formula (3), hereinafter referred to as 'Compound (3)', the sinocrassuloside VIII:

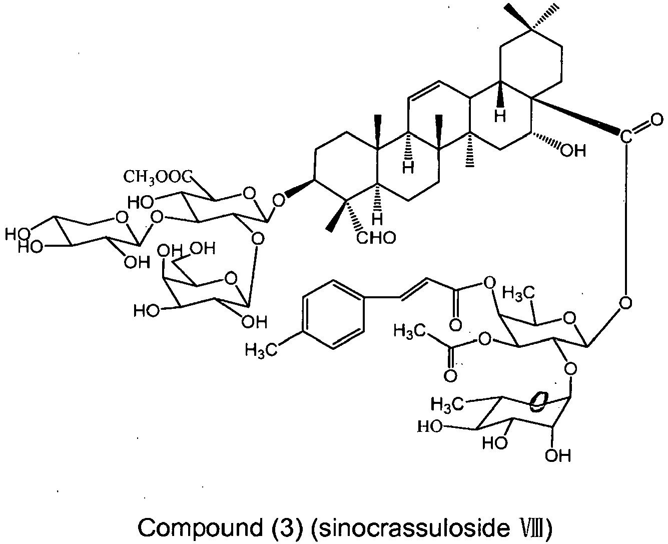

According to a further aspect, the said compound of *Wacao* saponin is represented by formula (4), hereinafter referred to as 'Compound (4)', the sinocrassuloside IX :

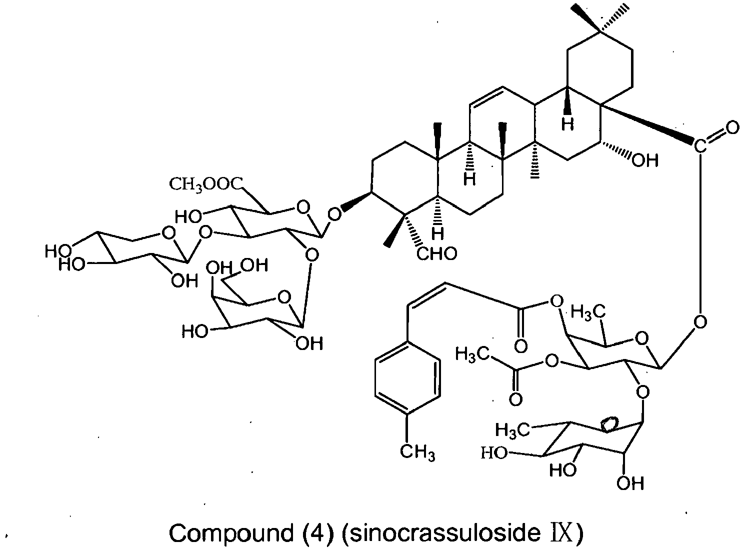

According to a further aspect, the said compound of *Wacao* saponins is represented by formula (5), hereinafter referred to as 'Compound (5)', the sinocrassuloside X:

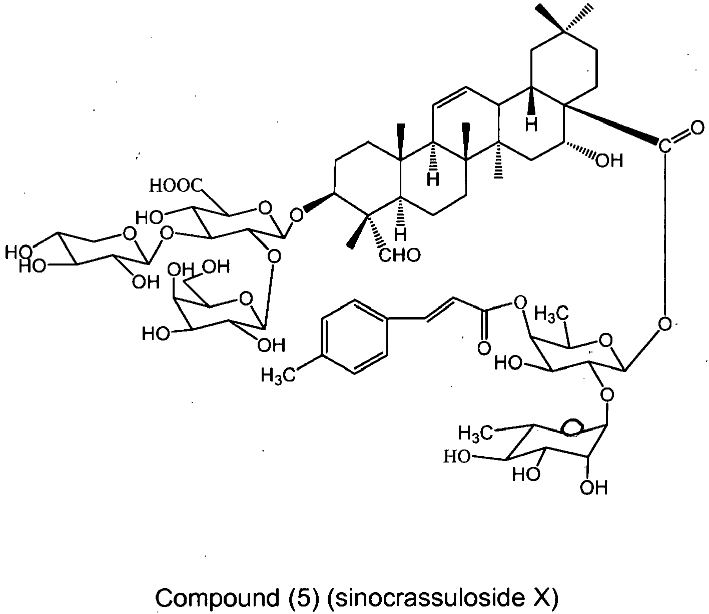

According to a further aspect, the said compound of *Wacao* saponin is represented by formula (6), hereinafter referred to as 'Compound (6)', the sinocrassuloside XI :

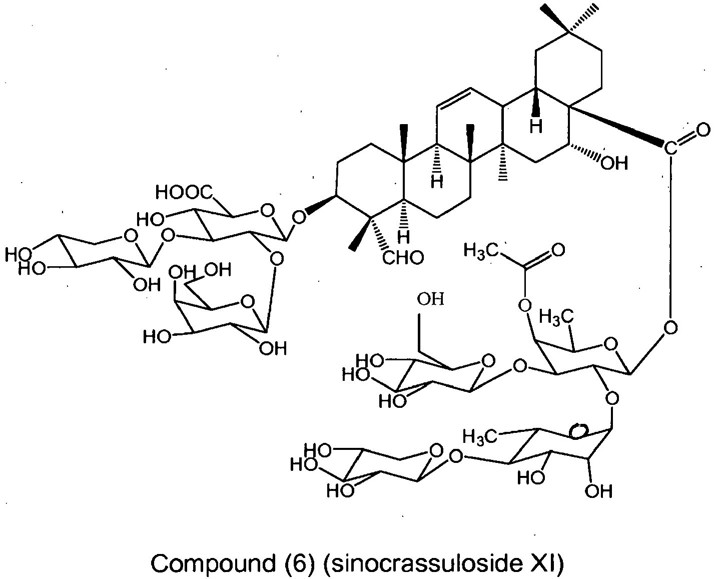

According to a further aspect, the-said compound of *Wacao* saponin is represented by formula (7), hereinafter referred to as 'Compound (7)', the sinocrassuloside XII:

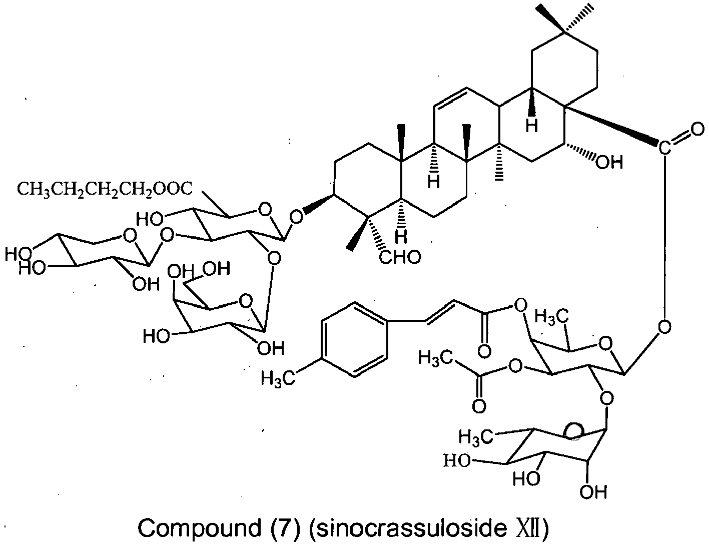

According to a further aspect, the said compound of *Wacao* saponin is represented by formula (8), hereinafter referred to as 'Compound (8)', the sinocrassuloside XIII:

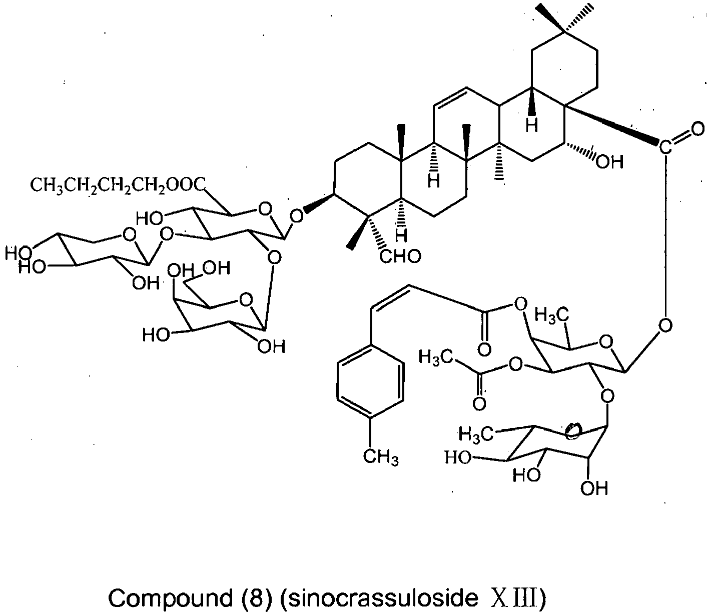

According to a further aspect, the said compounds of *Wacao* saponins may be used alone, a mixture of two or more, or mixed with other auxiliary materials into preparations which herein defined include injections, topical solutions, ointments, pastes, patches, drops, mouthwash, suppositories, sublingual tablets, paste film, aerosols, effervescent tablets and pills.

According to a further aspect, the said injections include intravenous injection, intramuscular injection, subcutaneous injection, intradermal injection, and intraperitoneal injection and the like.

According to a further aspect, the said topical solution refers to lotion or liniment.

According to a further aspect, the said ointment herein defined refers to ointment or plaster.

According to a further aspect, the said drops refer to eye drops or nose drops.

Still according to a further aspect, the said compounds of *Wacao* saponins may be used alone, a mixture of two or more, or mixed with other auxiliary materials for manufacturing medicaments, foodstuffs and drinks with the anti-diabetic activity.

### Beneficial Effects of the Invention:

The present invention provides an application of hypoglycemic drugs made from triterpenoid saponins, especially for the compounds comprising the mother nucleus structure of sinocrassuloside and the combination of above compounds. These compounds have the significant anti-diabetic activity, and can effectively lower the blood glucose and / or improve the body's glucose tolerance.

The compounds of *Wacao* saponins, their analogues and combinations, particularly the one derived from the mother nucleus structure of sinocrassuloside, have the obvious stronger hypoglycemic effects compared with hypoglycemic compounds from other reported plants.

The compounds of *Wacao* saponins, their analogues and combinations, particularly the one derived from the mother nucleus structure of sinocrassuloside, have the features of novel chemical skeleton structure compare to the present hypoglycemic drugs, simple preparation process, low pollution in production, stronger hypoglycemic effects and lower side effects.

The compounds of *Wacao* saponins, their analogues and combinations, particularly the one derived from the mother nucleus structure of sinocrassuloside, are the only chemical drug derived from natural extracts of plants that are able to compete with insulin in hypoglycemic effects.

### Brief Description of the Figures

Figure 1: Effects of *Wacao* saponins on glycemia in KK^{Ay} mice;
Figure 2: Effects of *Wacao* saponins on body weight in KK^{Ay} mice;
Figure 3: Effects of *Wacao* saponins on food intake in KK^{Ay} mice;
Figure 4: Effects of *Wacao* saponins on water intake in KK^{Ay} mice;
Figure 5: Effects of *Wacao* saponins on glycemia in KK^{Ay} mice;
Figure 6: Effects of *Wacao* saponins on maintaining time of anti-hyperglycemia after withdrawal in KK^{Ay} mice;
Figure 7:Effects of *Wacao* saponins on serum insulin content in KK^{Ay} mice;
Figure 8: Effects of *Wacao* saponins on ISI in KK^{Ay} mice;
Figure 9:Effects of *Wacao* saponins on hepatic glycogen in KK^{Ay} mice;
Figure 10: Effects of *Wacao* saponins on muscle glycogen in KK^{Ay} mice;
Figure 11:Effects of *Wacao* saponins on body weight in normal ICR mice;
Figure 12:Effects of *Wacao* saponins on blood glucose in normal ICR mice;
Figure 13:Effects of *Wacao* saponins on blood glucose in T2DM rats;
Figure 14:Effects of *Wacao* saponins on OGTT in T2DM rats;
Figure 15: Effects of *Wacao* saponins on hepatic glycogen in T2DM rats;
Figure 16: Effects of *Wacao* saponins on muscle glycogen in T2DM rats;
Figure 17: Effects of *Wacao* saponins on GSP content in T2DM rats;

Note: * *vs.* Control group, *p*<0.05; ** *vs.* Control group, *p*<0.01; ^{#}*vs.* Model group, p<0.05; *^{##}vs.* Model group, p<0.01.

### Experimental Examples

Hereinafter, the principles and features of this invention will be illustrated by reference to examples.

### Example 1

### Extraction, Separation, Purification and Structural Identification of Wacao saponins

The roots of *Silene viscidula* were dried and grinded into powders. 21 kg powders were extracted with 95 % and 70% ethanol three times under reflux and acquired 7 kg extracts. The following separation and purification method is according to the literature (J. Zhao, Norio Nakamura, Masao Hattori. New triterpenoid saponins from the roots of sinocrassula asclepiadea [J]. Pharmaceutical Society of Japan, 2004, 52(2):230-237). Six compounds were isolated and identified as sinocrassuloside VI, sinocrassuloside VII, sinocrassuloside VIII, sinocrassuloside IX, sinocrassuloside XII, and sinocrassuloside XIII. Among them, sinocrassuloside VI and sinocrassuloside VII, sinocrassuloside VIII and sinocrassuloside IX, sinocrassuloside XII and sinocrassuloside X III are the three pairs of cis-trans isomers.

Compound (1) and Compound (2) were obtained as white amorphous powder, ESI-MS (m/z): 1473.2 [M + Na]⁺, 1449.7[M-H]⁻; ¹H-NMR and ¹³C-NMR: Table1. The ESI-MS (m/z) of Compound (1) and (2) showed a molecular ion at m/z 1450 corresponding to the same molecular formula C₇₁H₁₀₂O₃₁. It was unambiguously identified as sinocrassuloside VI and sinocrassuloside VII on the basis of its ¹H-NMR and ¹³C -NMR spectral data (Table 1).

Compound (3) and (4) were obtained as white amorphous powder, ESI-MS (m/z): 1487.2 [M + Na]⁺, 1499.7 [M + Cl]⁻, 1463.8 [M-H]-. ¹H-NMR and ¹³C-NMR: Table1. The ESI-MS (m/z) of Compound (3) and (4) showed a molecularion at m/z 1464 corresponding to the same molecular formula C₇₂H₁₀₄O₃₁. It was unambiguously identified as sinocrassuloside VIII and sinocrassuloside IV on the basis of its ¹H-NMR and ¹³C -NMR spectral data (Table1).

Compound (7) and (8) were obtained as white amorphous powder, ESI-MS(m/z): 1529.3[M+Na]⁺, 1541.8[M+Cl]⁻,1505.6[M-H]⁻; ¹H-NMR and ¹³C-NMR: Table1. The ESI-MS (m/z) of Compound (7) and (8) showed the same molecular formula C₇₅H₁₁₀O₃₁ It was unambiguously identified as sinocrassuloside XII and sinocrassuloside XIII on the basis of its ¹H-NMR and ¹³C -NMR spectral data (Table 1).

**Table 1 ¹H-NMR and ¹³C-NMR data of Compounds (1), (2), (3), (4), (7) and (8)**

| | Compound (1) δHmult (J in Hz)^{a}) δC^{b}) | | Compound (2) δHmult (J in Hz)^{a}) δC^{b}) | | Compound (3) δHmult (J in Hz)^{a}) δC^{b}) | | Compound (4) δHmult (J in Hz)^{a})δC^{b}) | | Compound (7) δHmult (J in Hz)^{a}) δC^{b}) | | Compound (8) δHmult (J in Hz)^{a}) δC^{b}) | |
|---|---|---|---|---|---|---|---|---|---|---|---|---|
| The aglycone moiety | | | | | | | | | | | | |
| 1 | 0.81,1.37 | 37.8 | 0.82,1.37 | 37.8 | 0.85,1.39 | 37.8 | 0.85,1.39 | 37.8 | 0.86,1.40 | 37.7 | 0.86,1.40 | 37.7 |
| 2 | 1.82,2.09 | 24.8 | 1.82,2.09 | 24.8 | 1.81,2.01 | 24.7 | 1.81,2.01 | 24.7 | 1.83,2.02 | 24.9 | 1.82,2.029 | 24.9 |
| 3 | 3.94(7.8) | 84.2 | 3.94 | 84.2 | 4.09 | 84 | 4.09 | 84 | 4.1 | 84 | 4.1 | 84.1 |
| 4 | | 54.3 | | 54.3 | | 54.3 | | 55.3 | | 54.2 | | 54.2 |
| 5 | 1.35 | 48.5 | 1.35 | 48.5 | 1.36 | 48.5 | 1.36 | 48.5 | 1.37 | 48.4 | 1.37 | 48.4 |
| 6 | 0.88,1.37 | 20.2 | 0.88,1.37 | 20.2 | 0.89,1.36 | 20.2 | 0.89,1.36 | 20.2 | 0.89,1.38 | 20.3 | 0.89,1.38 | 20.2 |
| 7 | 1.5 | 32.2 | 1.5 | 32.2 | 1.51 | 32.1 | 1.51 | 32.1 | 1.52 | 32.1 | 1.52 | 32 |
| 8 | | 40.5 | | 40.3 | | 40.4 | | 40.4 | | 40.4 | | 40.3 |
| 9 | 1.78 | 46.3 | 1.78 | 46.3 | 1.78 | 46.8 | 1.76 | 46.8 | 1.78 | 46.7 | 1.77 | 46.7 |
| 10 | | 35.3 | | 35.3 | | 35.8 | | 35.9 | | 35.9 | | 35.8 |
| 11 | 1.91 | 23.3 | 1.91 | 23.3 | 1.91 | 23.3 | 1.91 | 23.3 | 1.92 | 23.2 | 1.92 | 23.2 |
| 12 | 5.57 brs | 121.8 | 5.57 br s | 121.8 | 5.60 br s | 121.8 | 5.60 br s | 121.8 | 5.61 brs | 122 | 5.61 br s | 122 |
| 13 | | 143.6 | | 143.5 | | 143.6 | | 143.6 | | 143.7 | | 143.6 |
| 14 | | 41.5 | | 41.5 | | 41.5 | | 41.5 | | 41.6 | | 41.7 |
| 15 | 1.90,2.18 | 35.8 | 1.90,2.16 | 35.9 | 1.95,2.18 | 35.8 | 1.91,2.18 | 35.9 | 1.96,2.20 | 35.9 | 1.96,2.20 | 36 |
| 16 | 5.20 brs | 73 | 5.17br s | 73 | 5.22 br s | 73.1 | 5.20 br s | 73.1 | 5.23 brs | 73.1 | 5.19br s | 73.2 |
| 17 | | 49 | | 49 | | 48.5 | | 48.5 | | 48.5 | | 48.5 |
| 18 | 3.39 d (13.8) | 40.9 | 3.38 | 40.9 | 3.39 d (14.0) | 40.9 | 3.39 d(14.0) | 40.9 | 3.40 d (14.0) | 40.9 | 3.4 | 40.9 |
| 19 | 1.34,2.74 t (14.0) | 47.9 | 1.34,2.74 t | 47.9 | 1.34,2.75 t (14.4) | 47.9 | 1.36,2.75 t(14.4) | 47.9 | 1.36,2.76 t (14.4) | 48 | 1.36,2.76 t (14.4) | 48 |
| 20 | | 30.6 | | 30.5 | | 30.5 | | 30.6 | | 30.5 | | 30.5 |
| 21 | 1.30,2.40 | 35.2 | 1.32,2.41 | 35.2 | 1.30,2.41 | 35.2 | 1.32,2.41 | 35.3 | 1.30,2.40 | 35.3 | 1.32,2.41 | 35.3 |
| 22 | 2.22,2.39 | 32.2 | 2.20,2.38 | 32.2 | 2.22,2.40 | 32.1 | 2.20,2.40 | 32.1 | 2.23,2.40 | 32 | 2.23,2.40 | 32 |
| 23 | 9.84 s | 210.5 | 9.84s | 210.5 | 9.86s | 210.5 | 9.86s | 210.5 | 9.88 s | 210.6 | 9.88s | 210.6 |
| 24 | 1.39 s | 10.7 | 1.39 s | 10.7 | 1.39 s | 10.7 | 1.40 s | 10.7 | 1.41 s | 10.7 | 1.41 s | 10.7 |
| 25 | 0.79 s | 15.8 | 0.84 s | 15.8 | 0.84 s | 15.8 | 0.88 s | 15.8 | 0.88 s | 15.7 | 0.88 s | 15.8 |
| 26 | 1.03s | 17.1 | 1.04 s | 17.1 | 1.07 s | 17.1 | 1.07 s | 17.2 | 1.08s | 17 | 1.08 s | 17 |
| 27 | 1.75 s | 26.7 | 1.77 s | 26.7 | 1.79 s | 26.7 | 1.76 s | 26.7 | 1.79 s | 26.8 | 1.79 s | 26.8 |
| 28 | | 175.1 | | 175.1 | | 175.1 | | 175.1 | | 175 | | 175.1 |
| 29 | 0.94s | 33.2 | 0.94s | 33.2 | 0.96 s | 33.2 | 0.98 s | 33.2 | 0.98 s | 33.3 | 0.99 s | 33.2 |
| 30 | 0.98 s | 24.7 | 0.99 s | 24.6 | 1.02 s | 24.6 | 1.02 s | 24.6 | 1.03s | 24.5 | 1.02 s | 24.4 |
| 3-*O*-β-D-Glucuronopyranosyl | | | | | | | | | | | | |
| 1' | 4.88 d (7.2) | 102.6 | 4.88 d (7.2) | 102.6 | 4.86 d (7.8) | 102.6 | 4.86 d (7.8) | 102.6 | 4.87 d (7.2) | 102.6 | 4.88 d (7.2) | 102.6 |
| 2' | 4.34 t (9.0) | 77.4 | 4.34 | 77.4 | 4.36 | 77.4 | 4.36 | 77.4 | 4.36 t (9.0) | 77.3 | 4.36 | 77.3 |
| 3' | 4.28 t (9.0) | 84.2 | 4.27 | 84.2 | 4.29 | 84.2 | 4.27 | 84.2 | 4.30 t (9.0) | 84.1 | 4.3 | 84.2 |
| 4' | 4.44 | 70.2 | 4.44 | 70.2 | 4.24 | 69.9 | 4.24 | 69.8 | 4.25 | 70.1 | 4.25 | 70 |
| 5' | 4.5 | 77 | 4.5 | 77 | 4.39 | 75.3 | 4.39 | 75.3 | 4.42 | 75.2 | 4.42 | 75.1 |
| 6' | | 170.4 | | 170.4 | | 169.5 | | 169.5 | | 179.8 | | 179.8 |
| 7' | | | | | 3.72 s | 52.4 | 3.72 s | 52.4 | 4.31 t (6.6) | 66.5 | 4.33 t (6.6) | 66.5 |
| 8' | | | | | | | | | 1.64 m | 28.3 | 1.67 m | 28.4 |
| 9' | | | | | | | | | 1.25 m | 22.5 | 1.28 m | 22.6 |
| 10' | | | | | | | | | 0.85 t (7.2) | 14.3 | 0.86 t (7.2) | 14.2 |
| 2'-O-β-D-Galactopyranosyl | | | | | | | | | | | | |
| 1" | 5.55 d (4.2) | 102.7 | 5.55 d (4.2) | 102.7 | 5.54 d (7.8) | 102.7 | 5.54 d (7.8) | 102.7 | 5.56 d (7.8) | 102.6 | 5.55 d (7.8) | 102.6 |
| 2" | 4.46 | 72 | 4.46 | 72 | 4.47 | 73 | 4.46 | 73 | 4.47 | 73.1 | 4.47 | 73.1 |
| 3" | 4.14 dd (7.8,3.6) | 6) 74 | 4.14 dd (7.8,3.6) | 74 | 4.14 dd | 74.9 | 4.14 dd | 74.9 | 4.15 dd (7.2,3.0) | 74.8 | 4.14 dd (7.2,3.0) | 74.9 |
| 4" | 4.55 | 68.9 | 4.55 | 68.9 | 4.57 | 68.5 | 4.57 | 68.5 | 4.56 | 68.4 | 4.56 | 68.4 |
| 5" | 4.02 | 75.3 | 4.02 | 75.3 | 4.02 | 77 | 4.02 | 77 | 4.02 | 77.1 | 4.02 | 77.1 |
| 6" | 4.43,4.50 | 60.2 | 4.42,4.50 | 60.2 | 4.44,4.52 | 60.3 | 4.44,4.51 | 60.3 | 4.45,4.51 | 60.2 | 4.45,4.51 | 60.2 |
| 3'-O-β-D-Xylopyranosyl | | | | | | | | | | | | |
| 1'" | 5.31 d (7.8) | 103.5 | 5.31 d(7.8) | 103.5 | 5.30 d (7.8) | 103.6 | 5.30 d (7.8) | 103.6 | 5.32d (7.8) | 103.6 | 5.32 d (7.8) | 103.6 |
| 2"' | 3.94 t (7.8) | 73.8 | 3.94 t (7.8) | 73.8 | 3.94 t (7.8) | 74.1 | 3.93 t (8.4) | 75.3 | 3.95 t (7.8) | 74.2 | 3.945(7.8) | 74.2 |
| 3"' | 4.08 | 77.4 | 4.08 | 77.4 | 4.08 | 77.4 | 4.07 | 77.4 | 4.09 | 77.4 | 4.09 | 77.4 |
| 4"' | 4.1 | 70 | 4.1 | 70 | 4.1 | 68.9 | 4.09 | 68.9 | 4.1 | 69.1 | 4.1 | 69.1 |
| 5"' | 3.57,4.21 | 66.2 | 3.57,4.20 | 66.2 | 3.64,4.21 | 66.2 | 3.62,4.21 | 66.2 | 3.66,4.22 | 66.3 | 3.66,4.22 | 66.2 |
| 28-O-β-D-Fucopyranosyl | | | | | | | | | | | | |
| 1"" | 6.17 d (8.4) | 93.2 | 6.13d (8.4) | 93.1 | 6.19d (7.8) | 93.2 | 6.14 d (7.8) | 93.2 | 6.20 d (8.4) | 93.3 | 6.15d (8.4) | 93.2 |
| 2"" | 4.71 t (8.4) | 71.3 | 4.62 t (8.4) | 71.3 | 4.72t (9.6) | 70.5 | 4.62 t (9.0) | 70.6 | 4.73 t (9.0) | 70.5 | 4.70 t (9.0) | 70.5 |
| 3"" | 5.68 dd (9.3,4.8) | 73.1 | 5.68 dd (9.3,4.8) | 73.1 | 5.70 dd | 73.8 | 5.69dd | 73.8 | 5.72 dd | 73.9 | 5.72dd | 74 |
| 4"" | 5.75 | 70.1 | 5.75 | 70.1 | 5.75 | 69.9 | 5.77 | 69.8 | 5.76 | 69.8 | 5.76 | 69.8 |
| 5"" | 4.2 | 69.9 | 4.21 | 68.5 | 4.19 | 68.5 | 4.2 | 68.5 | 4.21 | 68.5 | 4.21 | 68.5 |
| 6"" | 1.22 d (6.6) | 16.1 | 1.19d(6.6) | 16.1 | 1.24 d (6.0) | 16.1 | 1.21 d (6.6) | 16.1 | 1.26 d (6.6) | 16.3 | 1.25 d (6.6) | 16.3 |
| 2""-O-α-L-Rhamnopyranosyl | | | | | | | | | | | | |
| 1""' | 5.76 s | 101.6 | 5.75s | 101.6 | 5.77 s | 101.7 | 5.75s | 101.7 | 5.78 s | 101.7 | 5.76s | 101.8 |
| 2""' | 4.52 | 70.5 | 4.52 | 70.5 | 4.54 | 70.2 | 4.53 | 70.2 | 4.54 | 70.4 | 4.54 | 70.3 |
| 3""' | 4.36 | 70.6 | 4.36 | 68.5 | 4.36 | 71.9 | 4.35 | 71.9 | 4.37 | 72 | 4.36 | 72 |
| 4""' | 4.23 | 71.9 | 4.23 | 71.9 | 4.24 | 72 | 4.24 | 72 | 4.24 | 72.2 | 4.24 | 72.2 |
| 5""' | 4.4 | 70 | 4.4 | 70 | 4.44 | 68.9 | 4.44 | 68.9 | 4.46 | 68.7 | 4.45 | 68.6 |
| 6""' | 1.62 d (6.0) | 18.4 | 1.62 d (6.0) | 18.4 | 1.65 d (6.6) | 18.4 | 1.65d (6.6) | 18.4 | 1.66 d (6.6) | 18.8 | 1.65 d (6.6) | 18.8 |
| The acetyl group | | | | | | | | | | | | |
| 1'""' | | 169.9 | | 169.8 | | 169.9 | | 169.9 | | 170.2 | | 170.1 |
| 2'""' | 2.00 s | 20.9 | 1.97s | 20.9 | 2.01 s | 20.9 | 2.00 s | 20.7 | 2.02 s | 21 | 2.00s | 21 |
| The para-methoxycinmamoyl group (MC) | | | | | | | | | | | | |
| 1'""" | | 166.8 | | 166 | | 166.8 | | 166.8 | | 166.8 | | 166.7 |
| 2'""" | 6.60 d (15.6) | 114.7 | 5.91 d (12.9) | 114.8 | 6.61d (16.2) | 114.7 | 5.93 d (13.2) | 114.8 | 6.62 d (15.6) | 114.9 | 5.96d (12.0) | 114.8 |
| 3'""" | 7.95 d (15.6) | 145.9 | 6.95 d(12.6) | 145.9 | 7.96 d(16.2) | 145.9 | 6.96 d (13.2) | 145.9 | 7.97 d (15.6) | 146 | 6.98 d (12.0) | 146 |
| 4'""" | | 126.8 | | 127.1 | | 126.9 | | 127.1 | | 127 | | 127.1 |
| 5'""" & 9'""" | 7.53 d (12.6) | 130.9 | 7.96d (9.0) | 132.7 | 7.54 d (10.2) | 130.9 | 7.99d (8.4) | 132.7 | 7.55d(10.8) | 131 | 7.98d (9.0) | 132.8 |
| 6'""" & 8'""" | 7.00 d (8.4) | 114 | 6.95d (9.0) | 114 | 7.02 d (9.0) | 114.8 | 6.97 d (9.0) | 114.8 | 7.03 d (9.0) | 114.9 | 6.94d (9.0) | 114.9 |
| 7'""" | | 161.7 | | 160.8 | | 161.7 | | 160.8 | | 161.8 | | 160.8 |
| *p*-OCH₃ | 3.67 s | 55.7 | 3.64 s | 55.7 | 3.68 s | 55.8 | 3.65s | 55.7 | *3.67* s | 55.9 | 3.68 s | 55.8 |

### Example 2

### Effects of Wacao saponins on reduction of glycemia in type 2 diabetes mellitus (T2DM) mice

### Animals and breeding

80 male KK^{Ay} mice (12 weeks of age, License NO. SCXK (BJ) 2009-0015, Beijing Huafukang Biology Technology Co. Ltd.) and 10 male normal C57BL/6 mice (12 weeks of age, License NO. SCXK (BJ) 2009-0015, Beijing Huafukang Biology Technology Co. Ltd.) were adaptively bred for one week, and then used in the pharmacological experiment.

The breeding conditions were shown in table 2.

**Table 2 The breeding conditions of the experimental animals**

| | |
|---|---|
| Temperature | 22°C±2°C |
| Lighting | 12hs light- dark cycle |

A 12h light- dark cycle means the alternate time of light and dark for 12hs, respectively.

The animals were housed individually in wood-chip-bedded plastic cages. Germ-free and high-fat diets (Beijing Huafukang Biology Technology Co. Ltd.) were used to feed the KK^{Ay} mice, and the standard laboratory chows were used to feed normal C57BL/6 mice. All the animals had free access to food and water.

### Experimental Design

80 KK^{Ay} mice were adaptively bred for 1 week, and then randomly assigned into 8 groups: Model group, Compound (1) group, Compound (2) group, Compound (3) group, Compound (4) group, Compound (7) group, Compound (8) group and metformin group, 10 mice per group. 10 normal C57BL/6 mice were used as the control group. KK^{Ay} mice were fed with the high-fat diet, and the control group was fed with the normal control diet. The control and model groups were hypodermically injected with sterile water, 6 medicated groups were hypodermically injected with 6 kinds of *Wacao* saponins, the Compounds (1), (2), (3), (4), (7) and (8), and the positive group (metformin group) was irrigated per oral. All groups were administrated at 9 am for 2 weeks. The administrating volume is 10 ml/kg BW and the administration dosage is shown in Table3.

All mice of the fasting tail blood glucose level were measured weekly using One Touch ULTRA Glucometer (Life Scan Johnson and Johnson, USA).

**Table 3 The administration and dosage of the animals**

| Groups | Drugs | Dosage (mg/kg BW) |
|---|---|---|
| Control group | sterile water | - |
| Model group | sterile water | - |
| Compound (1) group | Compound (1) | 2.0 |
| Compound (2) group | Compound (2) | 2.0 |
| Compound (3) group | Compound (3) | 2.0 |
| Compound (4) group | Compound (4) | 2.0 |
| Compound (7) group | Compound (7) | 2.0 |
| Compound (8) group | Compound (8) | 2.0 |
| Metformin group | Metformin | 500 |

### Medication

Each compound of *Wacao* saponins was dissolved into sterile water and sterilized with filtration for Sc. The metformin was dissolved into sterile water for Ig. The control and model group mice were injected with sterile water, Sc. All the animals were treated at 9 am for 2 weeks.

### Experimental Procedures

Overnight fasting tail blood glucose level was measured weekly using One Touch ULTRA Glucometer and measured for twice.

### Termination of Experiment

Each group was administrated for 2 weeks (14 days) and then the experiment was terminated.

### Statistical Analysis

Results are expressed as means ± SD. Statistical analysis were analyzed by using SPSS 10.0 software. Data were evaluated with analysis of variance.

### Results

Effects of *Wacao* saponins on glycemia in KK^{Ay} mice

The main purpose of this experiment is to observe and compare the effects of different compounds of *Wacao* saponins on glycemia in KK^{Ay} mice, and provide experimental basis for the following studies.

Before administration (week 0), the KK^{Ay} mice were randomly divided into 8 groups according to their levels of glycemia. The average glycemia level of KK^{Ay} mice in model and *Wacao* saponins administration groups was markedly higher than that of the normal control C57BL/6 mice, the KK^{Ay} mice can be used as standard diabetic model animals.

After administrated for 1 week, each compound of *Wacao* saponins showed glycemia down-regulating effect at different degrees: Compound (1) showed the best hypoglycemic activity (*p*<0.01 vs model group), and then followed by Compound (2), Compound (4), Compound (3) and Compound (7), and the Compound (8) showed the weakest hypoglycemic activity which has no significant difference compared with that of the model group.

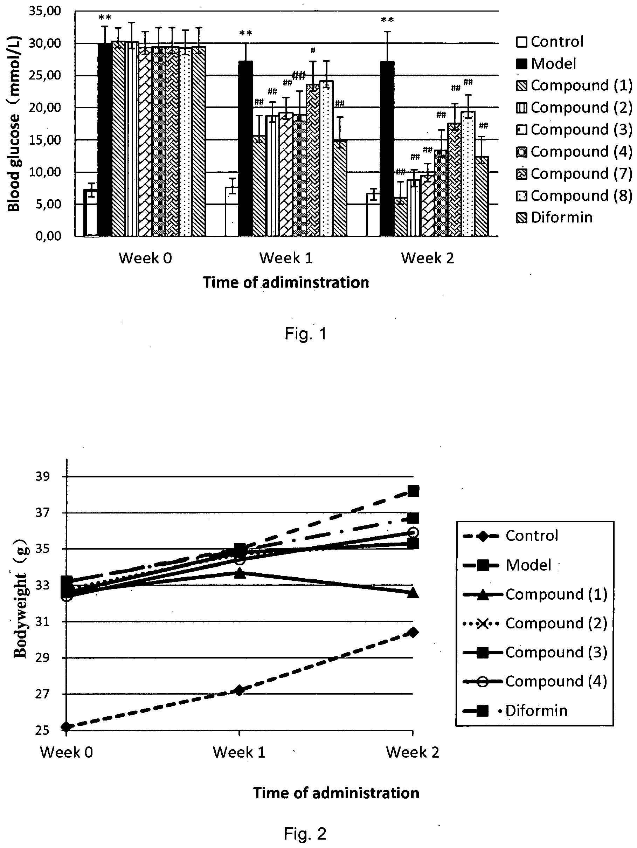

After the *Wacao* saponins-treated groups administrated for 2 weeks, the glycemic level kept on decreasing (*p*<0.01, *vs*. model group), and the tendency and degree of glycemia decreased in each compound treated group is the same as the week 1, as shown in Figure 1.

### Discussion

One of the key symptoms of diabetes is hyperglycemia. Constant hyperglycemia is harmful to patients' tissues, organs and cells, which may cause chronic complications such as diabetic nephropathy, diabetic foot, diabetic retinopathy and peripheral neuropathy, etc. So it is a critical step for the treatment of diabetes to reduce the level of blood glucose.

In this experiment, we compared the anti-hyperglycemia effects of different compounds with each other. It was indicated that Compounds (1) and (2) (cis-trans-isomers) had the strongest anti-hyperglycemia effect, followed by Compound (3) and (4) (cis-trans-isomers), and Compound (7), and the compound (8) showed no significant effect of anti-hyperglycemia compared with that of the other compounds. After one week's administration, Compound (8) showed only the trend of anti-hyperglycemia activity, but showed no statistical difference (*p*>0.05) compared with that of model group. Significant differences had not yet appeared even after two weeks' administration of Compound (8), which indicated that Compound (8) is far more inferior to that of the former 5 compounds in treating diabetes.

### Conclusion

*Wacao* saponins had strong anti-hyperglycemia effect, which are the ideal active ingredients for treating diabetes.

### Example 3

### The therapeutic effects of Wacao saponins in T2DM mice

### Animals and Breeding

60 male KK^{Ay} mice (12 weeks of age, License NO. SCXK (BJ) 2009-0015, Beijing Huafukang Biology Technology Co. Ltd.) and 10 normal male C57BL/6 mice (12 weeks of age, License NO. SCXK (BJ) 2009-0015, Beijing Huafukang Biology Technology Co. Ltd.) were adaptively bred for one week, and then used in the following experiment.

Animals were housed under the conditions as shown in table 4.

**Table 4 The breeding conditions of the experimental animals**

| | |
|---|---|
| Temperature | 22°C±2°C |
| lighting | 12hs light- dark cycle |

A 12h light- dark cycle means the alternate time of light and dark for 12hs, respectively.

The animals were housed individually in wood-chip-bedded plastic cages. Germ-free and high-fat diets (Beijing Huafukang Biology Technology Co. Ltd.) were used to feed the KK^{Ay} mice. All the animals had free access to food and water.

### Experimental Design

60 KK^{Ay} mice were adaptively bred for 1 week, and then randomly assigned into 6 groups: Model group, Compound (1) group, Compound (2) group, Compound (3) group, Compound (4) group, and metformin group, 10 mice per group. 10 normal C57BL/6 mice were used for the control group. KK^{Ay} mice were in a high-fat diet, and the control normal mice were in a normal control diet. The control and model groups were hypodermically injected with sterile water, the 6 medicated groups mice were subcutaneously injected with 6 kinds of *Wacao* saponins, (Compounds (1), (2), (3) and (4)) respectively, and the positive group was administered metformin by gavage. All mice were administrated at 9 am for 2 weeks and the administration volume is 10 ml/kg BW.

The body weight, food and water intake of each mouse was measured and recorded weekly throughout the course of the experiment. At the end of the experiment, collected the blood and separated serum, fasting blood glucose, insulin levels and insulin sensitivity index (ISI) were measured or calculated in each group. Liver, skeletal muscles of mice in each group were taken and weighed for detecting glycogen levels of the hepatic and muscles after mice being killed.

The animals were treated as follows:

**Table 5 The administration and dosage of the animals**

| Groups | Drugs | Dosage (mg/kg BW) |
|---|---|---|
| Control group | sterile water | - |
| Model group | sterile water | - |
| Compound (1)group | Compound (1) | 2.0 |
| Compound (2) group | Compound (2) | 2.0 |
| Compound (3) group | Compound (3) | 2.0 |
| Compound (4) group | Compound (4) | 2.0 |
| Metformin group | Metformin | 500 |

### Medication

Compound (1), (2), (3) and (4) were respectively dissolved into sterile water, and sterilized with filtration for Sc. to the medication groups. The metformin was dissolved into sterile water for Ig. to the positive control group. Control and model groups animals were injected with sterile water (10 ml/kg BW), Sc. All the animals were treated daily at 9 am for 2 weeks (14 days).

### Experimental Procedures

Measurement of food and water intake: 24 hours' food and water intake were measured by gravimetry once a week. It should be paid attention to recycling leakage of small forage into the feed box to ensure the accuracy of food intake.

Determination of body weight: The body weight of mice was measured and recorded once a week.

Blood detection: Blood glucose was measured weekly. Overnight fasting glycemia level was measured with One Touch ULTRA Glucometer for the tail blood test. At the end of the experiment, blood serum was collected for measuring levels of fasting blood glucose and insulin, and the insulin sensitivity index (ISI) was calculated. At the same time, hepatic tissues and skeletal muscles of mice were taken out to determine levels of hepatic and muscle glycogen by detection kits.

Termination of the experiment After administrating mice for 2 weeks (14 days), the experiment was terminated.

### Statistical Analysis

Results are shown as means ± SD. Statistical analysis data were analyzed by using SPSS 10.0 software. Data were evaluated with analysis of variance.

### Results

### Effects of Wacao saponins on body weight in KK^{Ay} mice

Experimental data showed a more significant increase in body weight of model group than that of in the control group (*p*<0.01), which was caused by obesity of KK^{Ay} mouse. In the meanwhile, the body weight growth in *Wacao* saponins-treated groups was obviously lower than that of model group, which indicated that *Wacao* saponins have a good activity of inhibiting the growth of body weight as shown in figure 2. Among them, Compound (1) showed the best effect of resistance on body weight growth in mice.

### Effects of Wacao saponins on food intake in KK^{Ay} mice

Results showed that level of food intake in the model group was significantly higher than that of the control group. The result is well in line with clinical diabetes 'polyphagia' symptom. After treated with different medication for one week, the food intake of the Compound (1) group and the positive metformin group decreased obviously compared with the model group (*p*<0.05). No statistical difference was found except the two groups above after one week treatment.

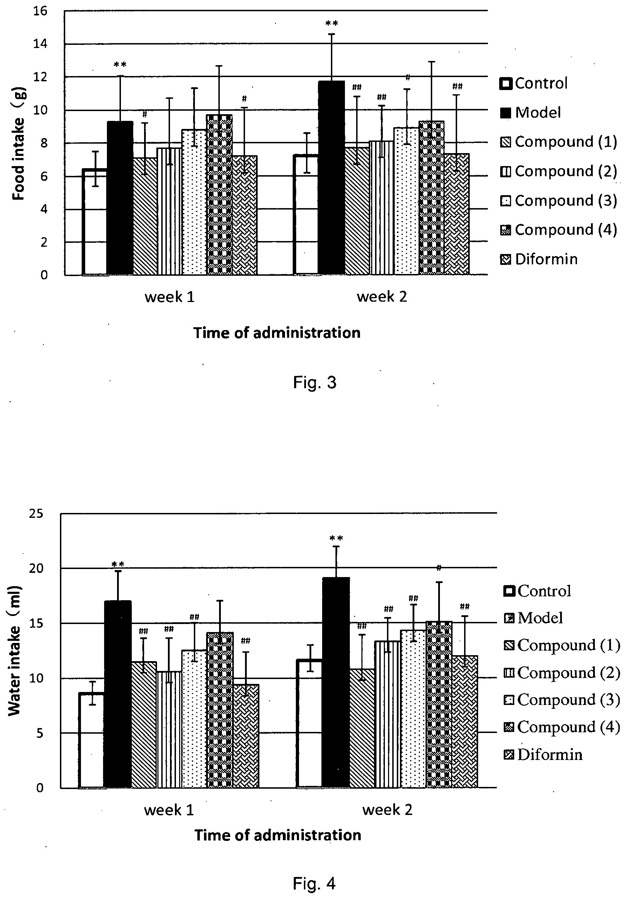

Two weeks after treatment, except the Compound (4) group, mice in all groups showed obvious reduction in food intake (*p*<0.05 or *p*<0.01, *vs.* model group), which exhibited a certain time - dependent manner, as shown in Figure 3.

### Effects of Wacao saponins on water intake in KK^{Ay} mice

Experimental data showed that level of water intake of the model group was much more than that of the control group, which was consistent with the clinical symptom of 'polydipsia' in diabetes patients. After treated with different medication for 1 or 2 weeks, water intake in each treatment group decreased significantly (*p*<0.05 or *p*<0.01, *vs.* model group), except Compound (4) group in week 1, as shown in Figure 4.

### Effects of Wacao saponins on glycemia in KK^{Ay} mice

This experiment is to observe the anti-hyperglycemia effects of *Wacao* saponins on KK^{Ay} mice during and after the period of drug treatment, respectively.

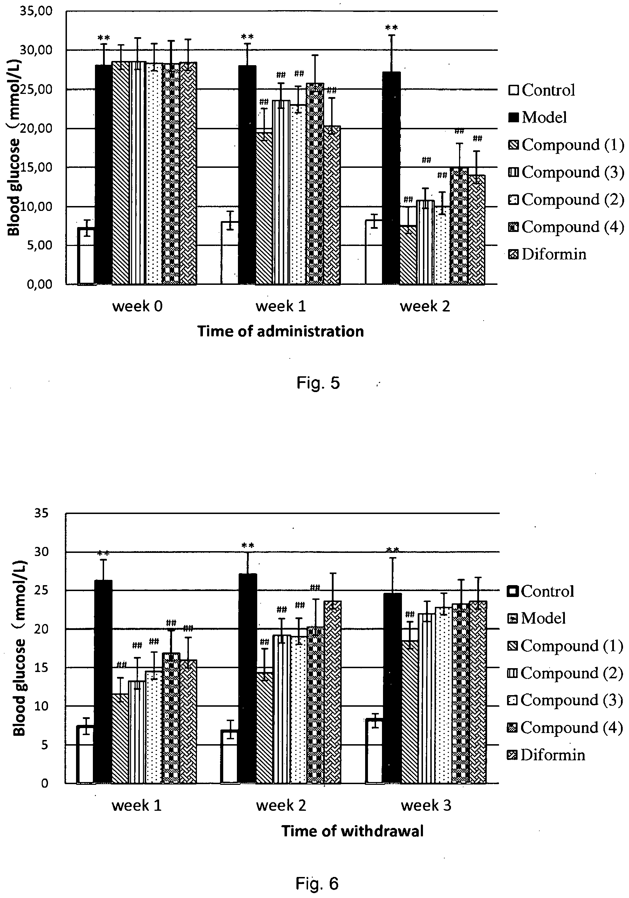

After one week of the medical intervention, each treatment group, except the Compound (4) group, showed a significantly decrease (*p*<0.01, *vs.* model group) in glycemia level. After two weeks of administration, the glycemia level in each compound treatment group decreased more obviously (*p*<0.01, *vs*. model group). Furthermore, results indicated that all treatment groups except Compound (4) had stronger anti-hyperglycemia effects than that of metformin, as shown in Figure 5.

Meanwhile, inventors also investigated the maintaining time of anti-hyperglycemia effects after withdrawal of *Wacao* saponins. Results showed that after the mice stopped taking medication, glycemic levels of all treated groups rebound gradually to different degrees. After 2 weeks of drug withdrawal, glycemic levels of metformin-treated group rebounded to a high level which showed no significant difference compared with that of the model group. It was indicated that the metformin's hypoglycemic effect disappeared after 2 weeks of stopping medication. Although each *Wacao* saponins-treated group's blood glucose level also slightly rebounded, it was still significantly lower than that of the untreated group (*p*<0.01). This hypoglycemic effect almost lasted for 3 weeks, which indicated that *Wacao* saponins have much longer duration in reducing hyperglycemia than metformin, as shown in Figure 6.

### Effects of Wacao saponins on blood insulin content and Insulin Sensitivity Index (ISI) in KK^{Ay} mice

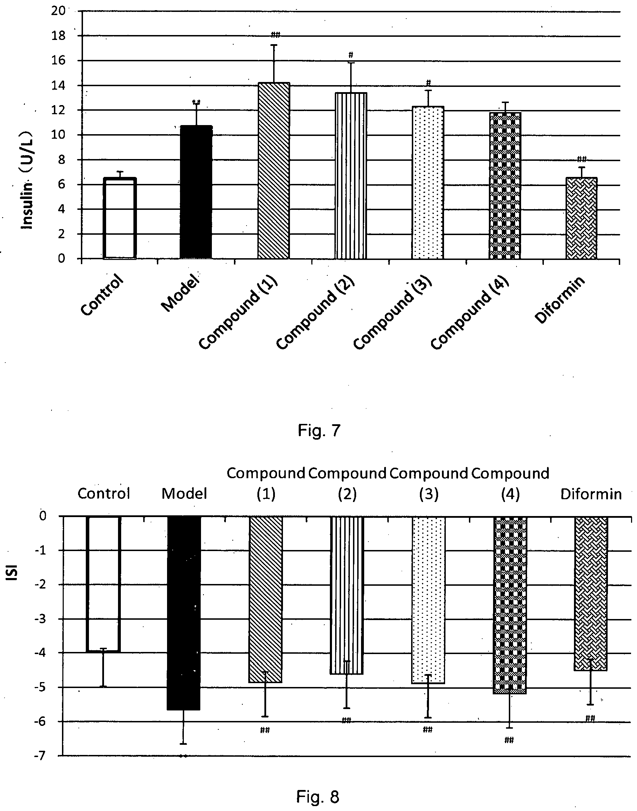

The experimental results showed that the insulin level of the model group was much higher than that of the control group (*p*<0.01) accompanied with higher glycemia level at the same time, which indicated a certain degree of insulin resistance (IR) appeared in the model group, which was consistent with T2DM syndrome. After mice being treated with different compounds of *Wacao* saponins, the insulin level of all groups except Compound (4) increased significantly (*p*<0.05 or *p*<0.01, *vs.* model group), which indicated that *Wacao* saponins promoted β cells to secrete insulin, as shown in Figure 7.

The ISI of the model group decreased remarkably, but it obviously elevated after treated with *Wacao* saponins (*p<*0.01, *vs.* model group), which indicated that *Wacao* saponins may improve the insulin sensitivity of animals or human, as shown in Figure 8.

Effect of *Wacao* saponins on the level of hepatic and muscle glycogen in KK^{Ay} mice The data demonstrated that the level of hepatic glycogen and muscle glycogen of KK^{Ay} mice significantly reduced compared with the normal mice (*p*<0.01). Results showed that all compounds of *Wacao* saponins had the obvious activities of increasing hepatic glycogen level (*p*<0.05 or *p*<0.01, *vs.* model group), but exhibited an incoordinate efficacy in increasing muscle glycogen level, Compounds (1) and (2) showed stronger efficacy in raising levels muscle glycogen (*p*<0.01, *vs.* model group), but Compound (3) and (4) showed a weaker efficacy in raising level of muscle glycogen which had no statistical significant difference compared with that of the model group.

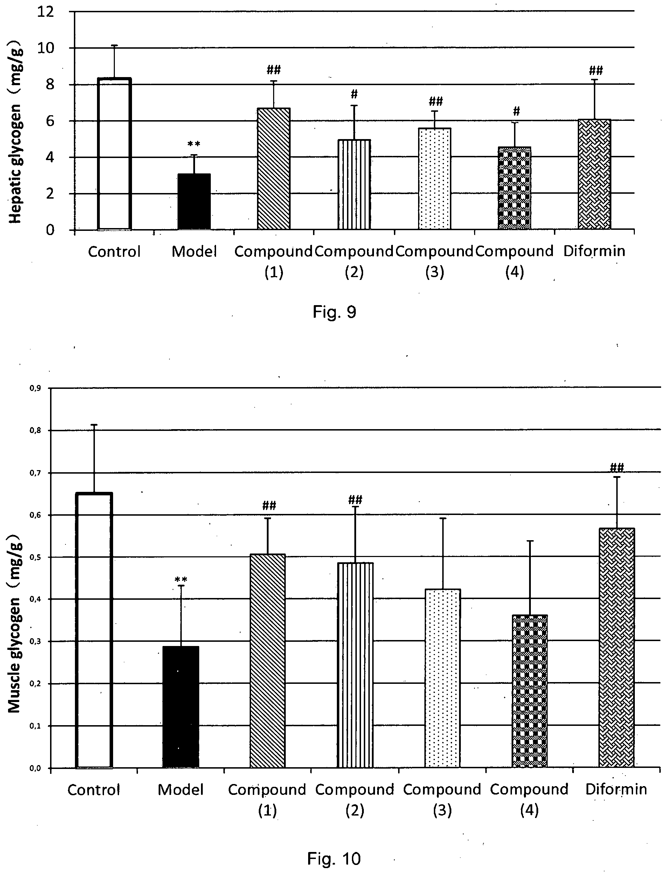

The experiment demonstrated that *Wacao* saponins can enhance the glycogen storage, especially for the hepatic glycogen. Among them, Compound (1) showed the strongest activity of the function. It was almost comparable to metformin, as shown in Figure 9 and 10.

### Discussion

The main symptom of diabetes is hyperglycemia, accompanied with such clinical manifestations as polydipsia, polyphagia, diuresis and athrepsy. Constant hyperglycemia will harm the body tissues, organs and cells, and lead to diabetic nephropathy, diabetic foot and eye ground, peripheral neuropathy and other chronic complications. Therefore, it is the primary goal for treating diabetes to reduce the body's glucose level. In addition to high blood sugar, Type 2 diabetes is often characterized by abnormal glucose tolerance, the drop of insulin sensitivity, lipid metabolism disorder and abnormal insulin tolerance. Laboratory tests show high levels of TC, TG and LDL, but low levels of HDL in T2DM patients.

The results of this experiment indicated that *Wacao* saponins could effectively and efficiently improve the symptoms of polydipsia, polyphagia of diabetic model mice, decrease the blood glucose, promote the secretion of insulin, increase ISI and the contents of hepatic and muscle glycogen. Among them, Compound (1) showed the best anti-diabetic effects characterized by good hypoglycemic effect and the increase in insulin sensitivity, which is similar to the positive drug of metformin. Furthermore, its hypoglycemic effect lasts longer than that of metformin, which can effectively prevent blood sugar from rebounding in a short period of time after stopping medication. These observations collectively demonstrate that the anti-diabetic activity of *Wacao* saponins may be related to such functions as promoting insulin secretion, enhancing insulin sensitivity and promoting reserves of peripheral blood sugar in body's tissues.

### Conclusion

*Wacao* saponins can improve the symptoms of polydipsia and polyphagia of diabetic model mice, effectively promote insulin secretion, increase ISI and enhance glycogen reserves, which indicated that *Wacao* saponins have a strong hypoglycemic effect with good druggability, and can be used to manufacture the ideal drugs in treating diabetes.

### Example 4

### Effects of Wacao saponins on reducing glycemia in normal ICR mice

This experiment is to investigate the effect of Compounds (1) and (2) on reducing glycemia in normal ICR mice.

### Animals and Breeding

40 male ICR mice (20-22g, License NO. SCXK (BJ) 2011-0004, Beijing SPF Experimental Animal Technology Co. Ltd.) were adaptively bred for one week, and then used in the following experiment.

Animals were housed under the following condition, as shown in Table 6

**Table 6 The breeding conditions**

| | |
|---|---|
| Temperature | 22°C±2°C |
| lighting | 12hs light- dark cycle |

A 12h light- dark cycle means the alternate time of light and dark for 12hs, respectively. Animals were housed individually in wood-chip-bedded plastic cages and fed the standard laboratory chow. All the animals had *ad libitum* access to food and water.

### Experimental Design

40 ICR mice were adaptively bred for 1 week, and then randomly assigned into 4 groups: Model, Compound (1), Compound (2) and metformin group, 10 mice per group. All the animals were fed with normal food. Mice in the control group were hypodermically injected with sterile water. The treated groups were subcutaneously injected with Compounds (1) and (2), respectively. And the positive group was administrated metformin by gavage. All the animals were administrated at 9 am for 2 weeks, and the dose volume is 10 ml/kg BW.

The body weight and fasting blood glucose were measured and recorded weekly.

The animals were treated as the following:

**Table 7 The administration and dosage of the animals**

| Groups | Drugs | Dosage (mg/kg BW) |
|---|---|---|
| Control group | sterile water | - |
| Compound (1) group | Compound (1) | 2.0 |
| Compound (2) group | Compound (2) | 2.0 |
| Metformin group | Metformin | 500 |

### Medication

Compound (1) and (2) were respectively dissolved into sterile water, and sterilized with filtration for Sc. The metformin was dissolved into sterile water for Ig. to the positive control group. Mice in the control group were injected with sterile water (10 ml/kg BW), Sc. All the animals were treated at 9 am for 2 weeks (14 days).

### Experimental Procedures

Determination of body weight: Body weights of animals were weighed and recorded once a week.

Blood detection: Blood glucose was measured weekly. Overnight fasting glycemia was measured by One Touch ULTRA Glucometer (LifeScan Johnson and Johnson, USA) for the tail blood test.

End of the experiment: After mice being administrated for 2 weeks (14 days), the experiment was terminated.

### Statistical Analysis

Data were expressed as mean±SD. Statistical analysis data were analyzed by using SPSS 10.0 software. Data were evaluated with analysis of variance.

### Results

### Influence of Wacao saponins on body weight in normal ICR mice

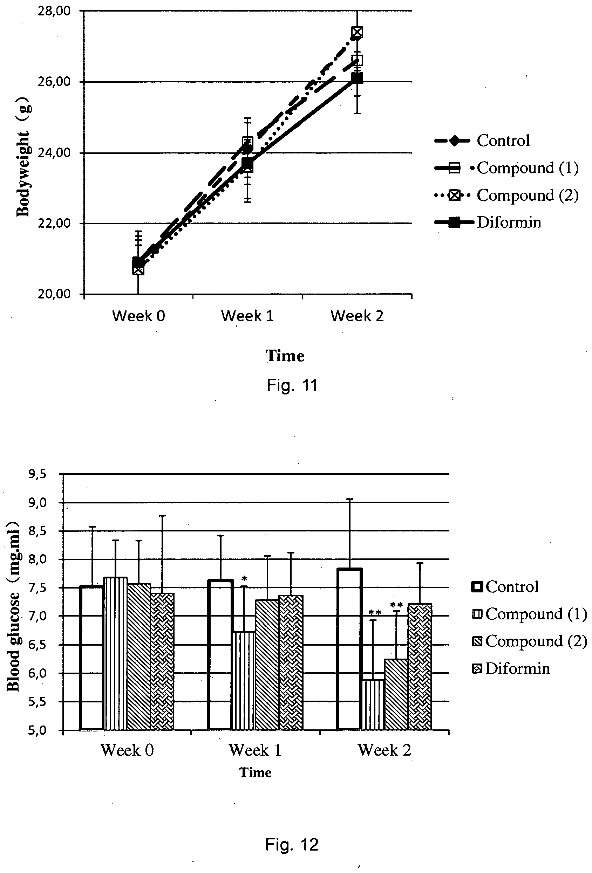

There was no significant difference found in body weight between the *Wacao* saponins-treated and the normal control groups, which indicated that *Wacao* saponins have no obvious impact on body weight of ICR mice, as shown in Figure11.

Effects of *Wacao* saponins on glycemia in normal ICR mice

After mice being administrated for 1 week, the glycemia level in each *Wacao* saponins treated groups was decreased more or less, but no significant difference was found except the Compound (1) group (*p*<0.05, vs. control group) compared with the control group. After mice being administrated for 2 weeks, the glycemia level of the Compounds (1) and (2) group decreased much obviously (*p*<0.01, *vs.* control group), which indicated a stronger effect on anti-hyperglycemia, as shown in Figure 12.

### Conclusion

Compound (1) and (2) can decrease the normal blood glucose of ICR mice, which indicated that *Wacao* saponins had obvious hypoglycemic effects on normal ICR mice as well.

### Example 5

### The therapeutic effects of Wacao saponins in experimental T2DM model rats

### Animals and Breeding

50 male SD rats (180-200g, License NO. SCXK (BJ) 2011-0004, Beijing SPF Experimental Animal Technology Co. Ltd.) were adaptively bred for 1 week, and then used in the pharmacological experiment.

Animals were housed under the conditions as shown in table 8.

**Table 8 The breeding conditions**

| | |
|---|---|
| Temperature | 22°C±2°C |
| lighting | 12hs light- dark cycle |

A 12h light- dark cycle means the alternate time of light and dark for 12hs, respectively.

Animals were housed individually in wood-chip-bedded plastic cages and fed a standard laboratory chow. All the animals had *ad libitum* access to food and water.

### Experimental design

50 SD rats were adaptively bred for 1 week, and then randomly assigned into 5 groups: The control group, model group, Compound (1) group, Compound (2) group and glucobay group (the positive group), 10 rats per group. All the animals were in a standard laboratory diet. Control and model group animals were hypodermically injected with sterile water, test compound treatment groups were subcutaneously injected with the Compounds (1) and (2), and the positive group was administrated glucobay solution by gavage. All the animals were administrated at 9 am for 2 weeks, and the dose volume is 10ml/kg BW.

The body weight and fasting blood glucose level of each rat was measured weekly.

The animals were treated as follows:

**Table 9 The administration and dosage**

| Groups | Drugs | Dosage (mg/kgBW) |
|---|---|---|
| Control group | sterile water | - |
| Model group | sterile water | - |
| Compound (1) group | Compound (1) | 4.0 |
| Compound (2) group | Compound (2) | 4.0 |
| Glucobay group | Glucobay | 20 |

### Medication

The Compounds (1) and (2) were dissolved into sterile water, and sterilized with filtration, Sc.. Glucobay was dissolved into sterile water, Ig. Animals in the control and model groups were injected with sterile water (10ml/kg BW), Sc. All the animals were treated at 9 am for 2 weeks (14 days).

### Experimental Procedures

### Preparation of T2DM animal model

All rats except the control group were administrated intragastrically with the high-fat emulsion (10ml/kg) and weighed the body weight weekly. After 4 weeks of administration, rats were intraperitoneally injected with streptozotocin (STZ, dissolved in citric acid-citrate sodium solution, pH 4.2, freshly prepared and used instantly, and store away from light) (30mg/kg). 4 days later, blood glucose level in rats was measured by tail blood test. Diabetes was defined as fasting blood glucose level>11.1 mmol/Lin in this experiment.

### Blood glucose measurement

The blood glucose level was measured before and after administration of the treatment compounds for 2 weeks, respectively. The animals were fasted overnight and tail blood samples were taken. Fasting glycemia was measured by using One Touch ULTRA Glucometer or kits.

### Oral Glucose Tolerance Test (OGTT)

OGTT was performed after administration for 2 weeks. Animals were fasted for 16hours and the fasting blood glucose level was measured prior to the start of the OGTT. Then animals were treated with 50% glucose water solution at rate of 5g/kg BW by gavage. Blood samples were collected and the blood glucose level was measured at 30, 60 and 120 minutes after the glucose load.

### Measurement of glycosylated serum protein (GSP) and glycogen level of liver and muscles

At the end of the experiment, the abdominal aorta blood was collected to determine GSP, and the liver and skeletal muscles were taken to detect glycogen.

### Termination of the experiment

The experiment was terminated after 2 weeks of compounds administration.

### Statistical Analysis

Data are expressed as mean±SD. Statistical analysis data were analyzed by using SPSS 10.0 software. Data were evaluated with analysis of variance.

### Results

### Effect of Wacao saponins on glycemia in T2DM rats

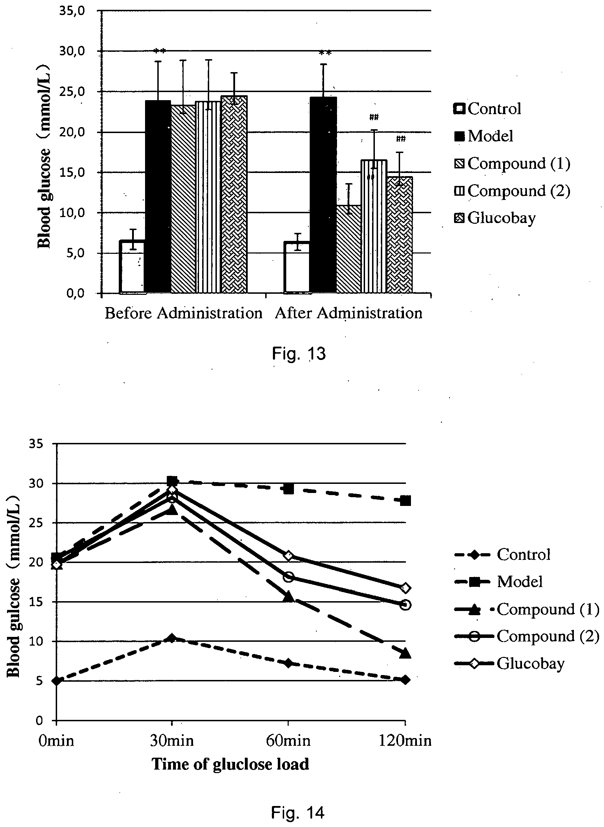

After mice being administrated for 2 weeks, the glycemia level of Compounds (1) and (2) groups was significantly decreased compared with the model group (*p*<0.01, *vs.* model group).The result indicated that Compounds (1) and (2) had a strong effect on anti-hyperglycemia. And such efficacy of Compound (1) was much stronger than that of the glucobay, as shown in Figure 13.

### Effect of Wacao saponins on OGTT in T2DM rats

30 minutes after the glucose load, all groups' glycemia level increased, but the normal control group increased inconspicuously. The model and *Wacao* saponins-treated groups increased dramatically. No statistical significant difference was observed between the model and treatment groups. Glycemia level of the control group started increasing at 30mins after glucose load, then continuously decreased to normal level at 120 mins. The glycemia level of the model group decreased slowly and maintained a higher level within 120 minutes after glucose load compared with the normal control group, which demonstrated that the glucose tolerance of the model group was impaired. By contrast, compounds-treated groups' glycemia level declined significantly compared with the model group (*p*<0.05 or *p*<0.01). Compounds (1) and (2) showed a similar effect on improving glucose tolerance to glucobay, as shown in Figure 14.

### Effect of Wacao saponins on the contents of GSP and glycogen of hepatic and muscle in T2DM rats

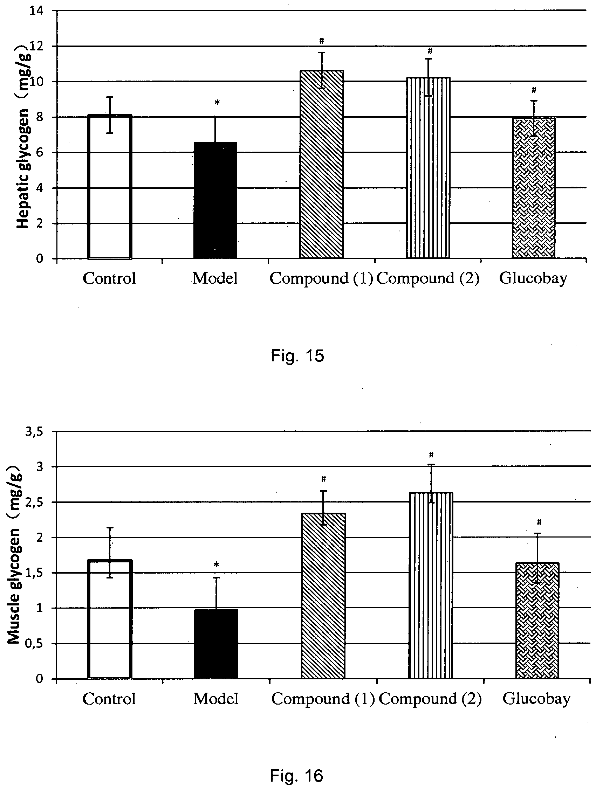

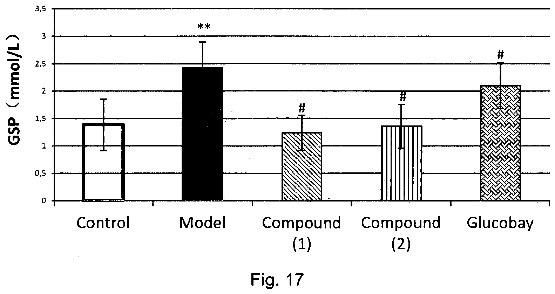

Data demonstrated that hepatic and muscle glycogen levels of T2DM rats decreased significantly (*p*<0.05), but the GSP level increased obviously (*p*<0.01) compared with the normal rats. After treatment, the hepatic and muscle glycogen levels of each treatment group significantly increased, but the GSP level obviously decreased at the same time. Compounds (1) and (2) showed a better effect on up-regulating of glycogen levels and down-regulating of GSP levels in the oral glucose tolerance test than glucobay, as shown in Figure 15, 16 and 17.

### Discussion

At present, it is a common method to prepare diabetes animal model by injecting overdose of streptozotocin (STZ) or alloxan, whose pathological mechanism is to selectively destroy pancreatic β cells and reduce the content of blood insulin, which leads to increase blood glucose level. The results reasonably account for the pathologic features of type 1 diabetes mellitus (T1DM). The features of the deficiency of insulin secretion caused by STZ or alloxan are quite different from the pathologic process and clinical features of the type 2 diabetes mellitus (T2DM). In this experiment, we used high-fat diet administration combining low pathologic dose injection of STZ to establish the T2DM animal model.

Data showed that glycemia level of model group was much higher than that of the normal control group, which demonstrated that T2DM model was successfully established. After treated with Compounds (1) and (2), the glycogen level decreased and the GSP level increased in the model animals. The results demonstrated that Compounds (1) and (2) had a good anti-hyperglycemia effect on T2DM and the efficacy was stronger than glucobay.

The experimental results demonstrated that hepatic and muscle's glycogen contents declined obviously in T2DM model rats compared with that of normal rats (p<0.05). Fortunately, Compounds (1) and (2) could obviously elevate hepatic and muscles glycogen level in T2DM model rats. And the increase in glycogen level was even higher than that of the normal rats, which indicated that Compounds (1) and (2) had a good effect on inhibiting glycogenolysis, increasing the glycogen level, and counteracting the hyperglycemia which can cause damage to liver and peripheral tissues. Such effect of the compounds is superior to that of glucobay.

GSP is a kind of high molecule ketoamine with stable chemical structure, which is formed when no enzymatic glycosylation action happens between glucose and serum protein molecule at high glucose status. As the halflife time of serum albumin is about 17 to 20 days, GSP level can reflect the average glycemia level in 2 or 3 weeks, which will not be invulnerable to glycemia content. The results showed that GSP level rose markedly in T2DM model group, but Compounds (1) and (2) down-regulated the GSP level significantly, whose effects are stronger than glucobay.

### Conclusion

Compounds (1) and (2) have good efficacies of reducing blood glucose, improving glucose tolerance, increasing liver and muscle's glycogen level, and decreasing serum GSP content. All in all, these results indicated that Compounds (1) and (2) were certain strong hypoglycemic ingredients which could be used to manufacture anti-diabetic medicaments.

## Claims

1. A compound of *Wacao* saponins for use in the treatment of diabetes, wherein said compound is selected from the compounds having following formula:

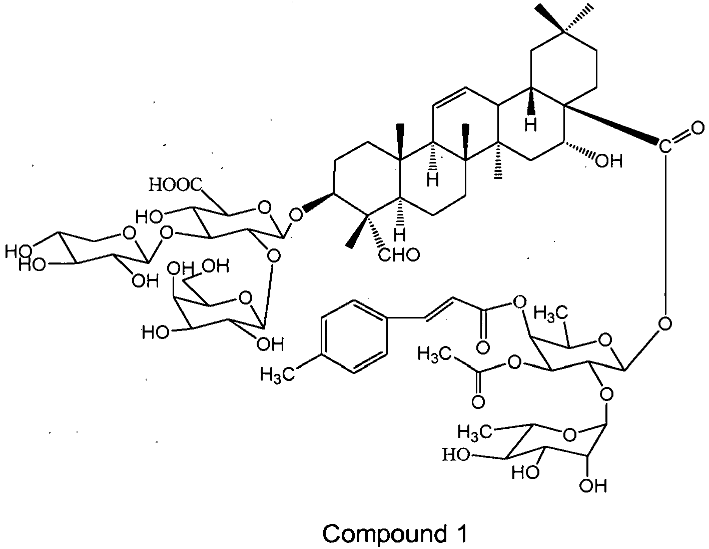

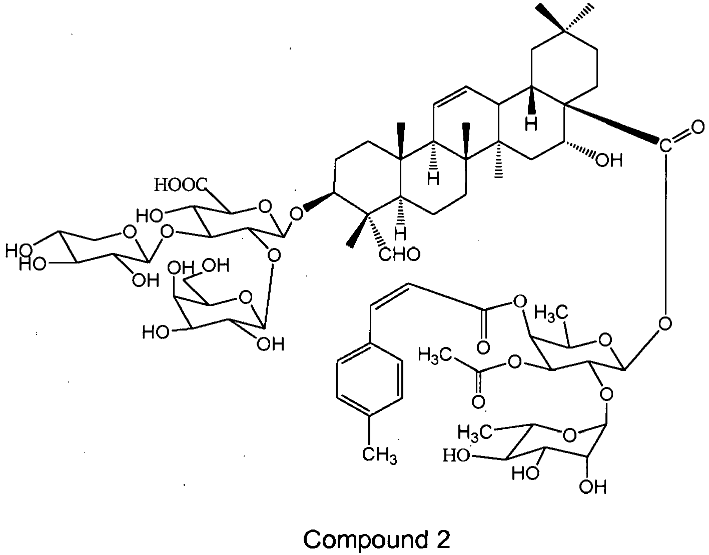

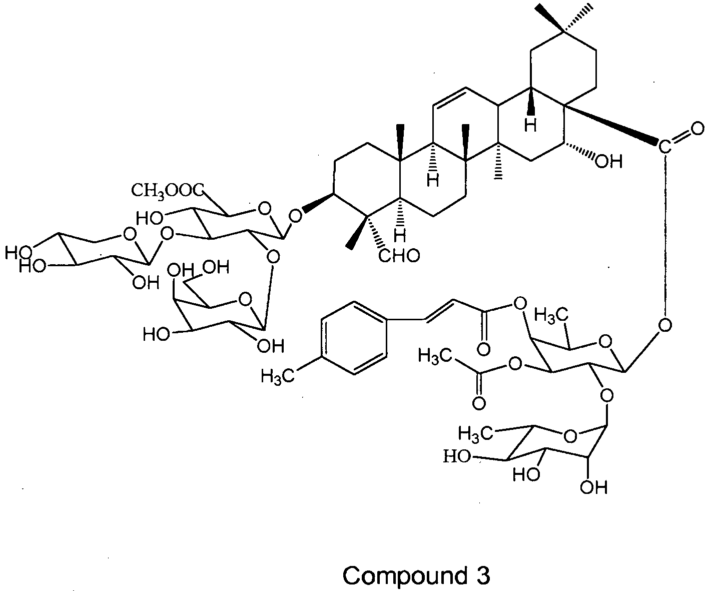

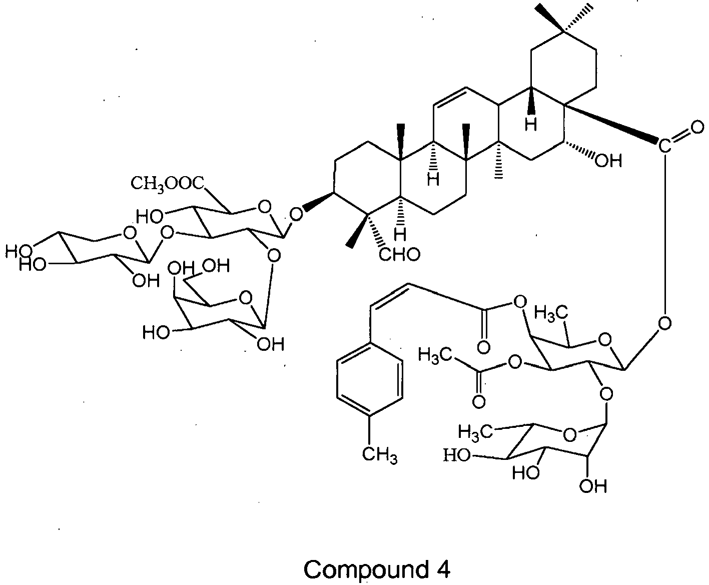

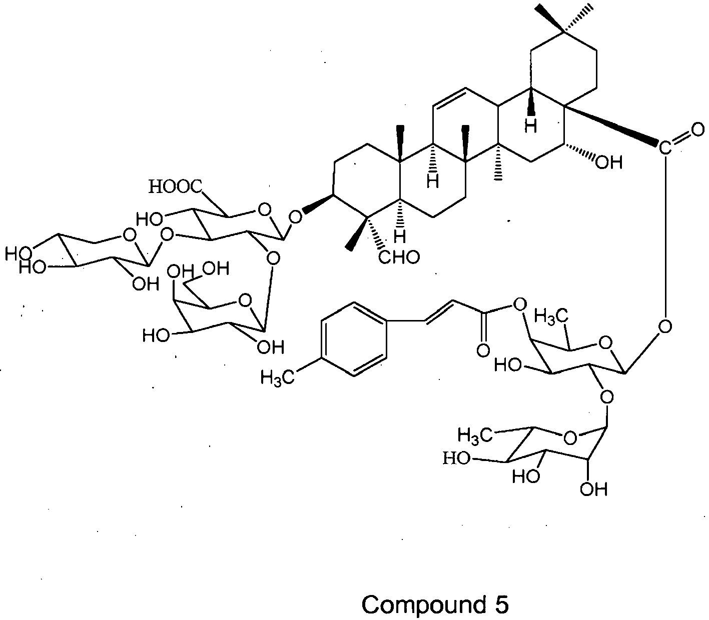

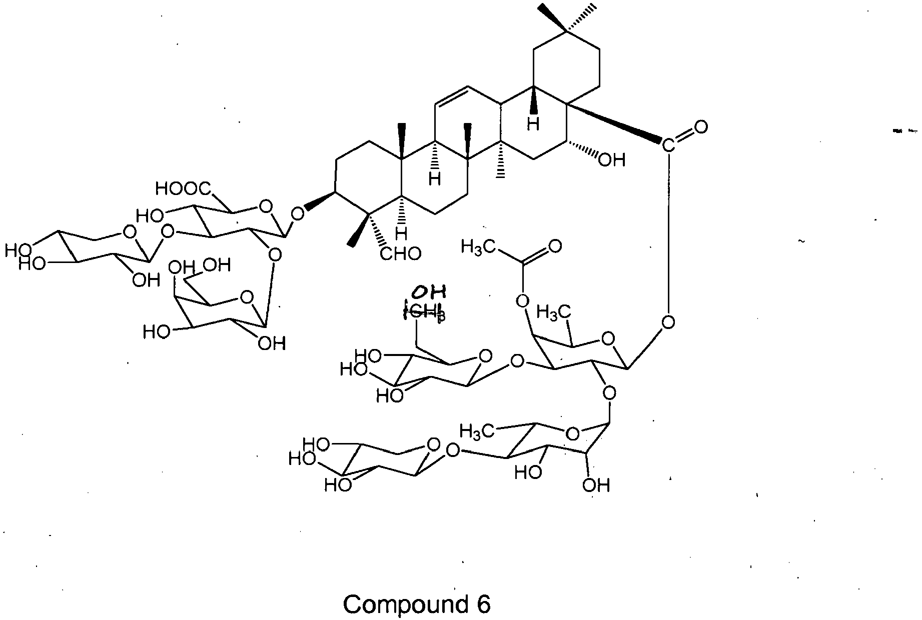

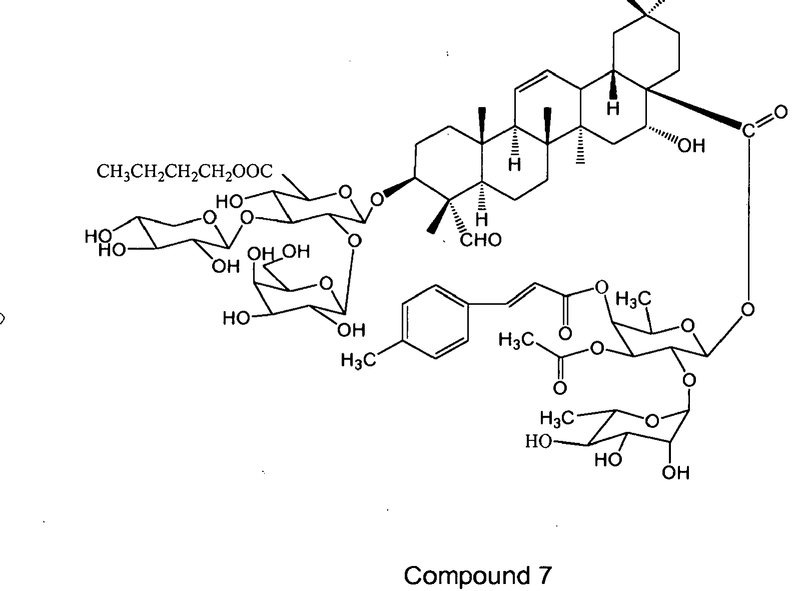

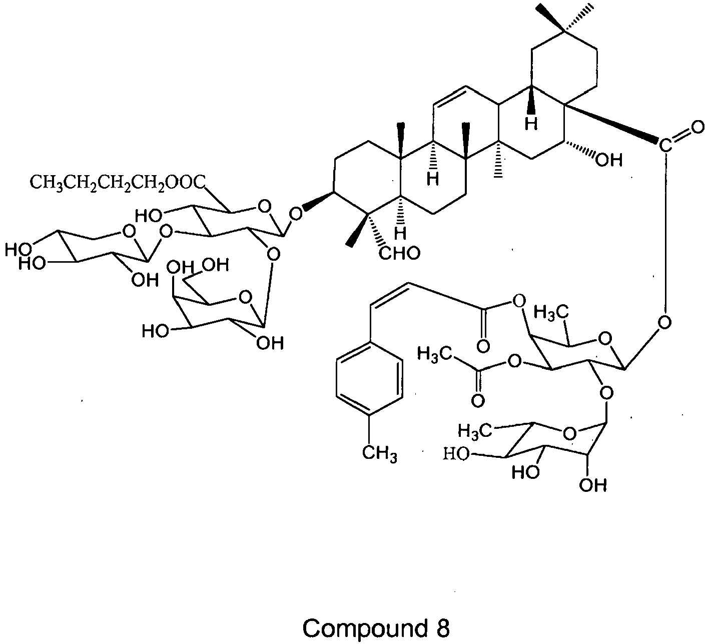

2. The compounds for use according to claim 1, wherein said compounds are used alone, in a mixture of two or more, or mixed with other materials to make into agents as injections, topical solutions, ointments, pastes, patches, drops, gargles, suppositories, sublingual tablets, paste films, aerosol agents, effervescent tablets, and pills agents.

## Patentansprüche

1. Verbindung aus Wacao-Saponinen zur Verwendung bei der Behandlung von Diabetes, wobei die Verbindung aus den Verbindungen mit den folgenden Formeln ausgewählt ist:

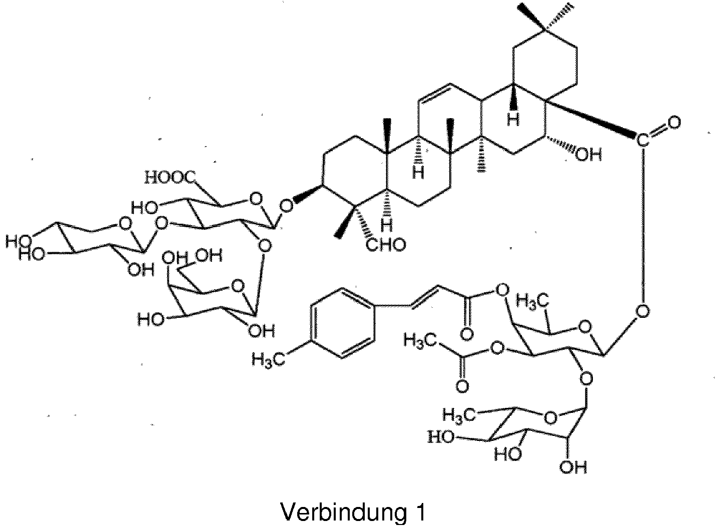

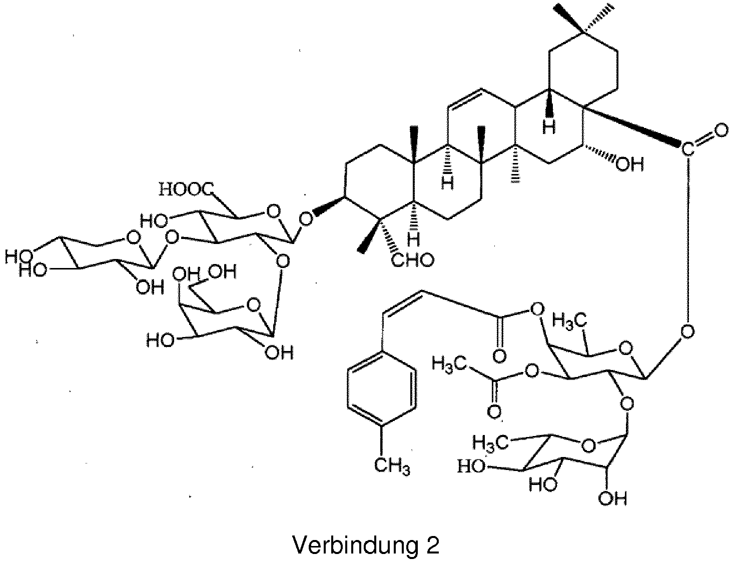

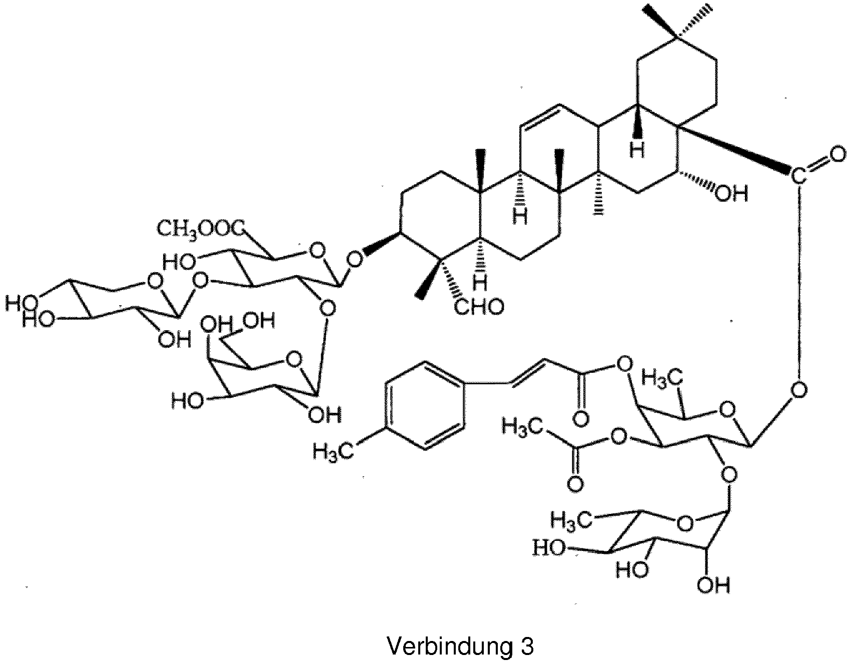

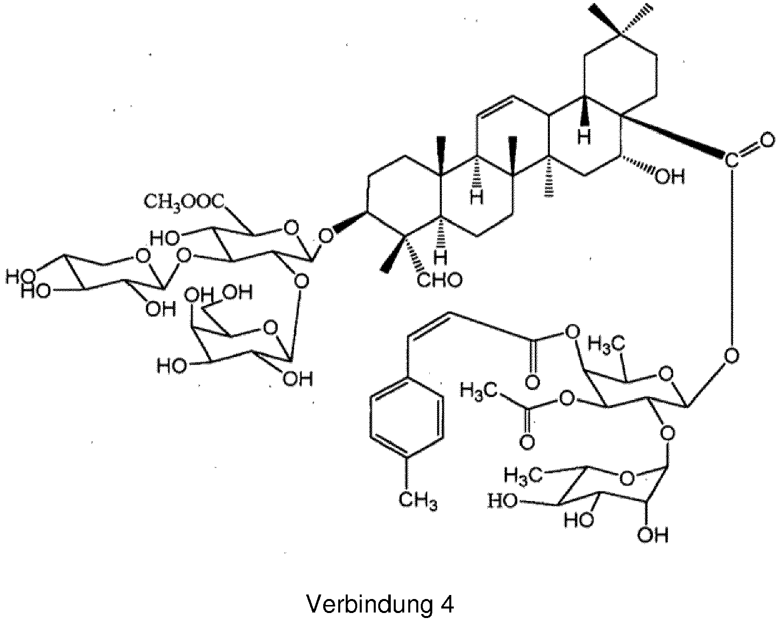

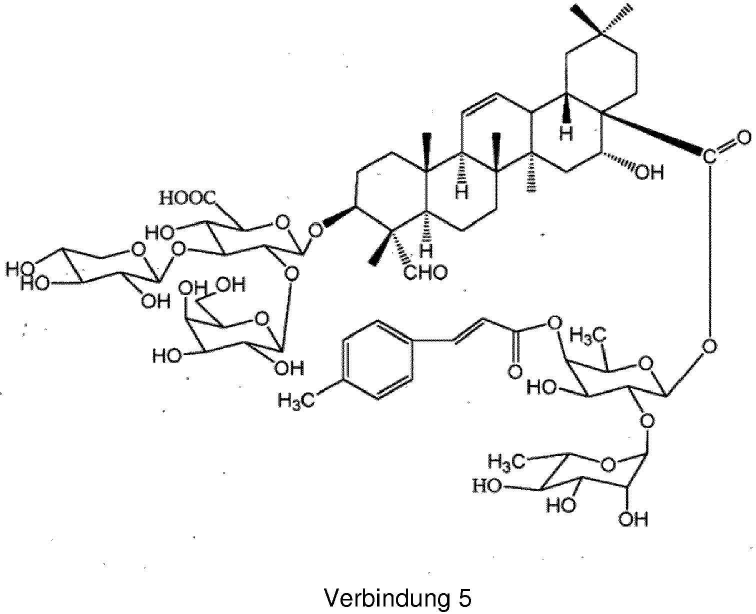

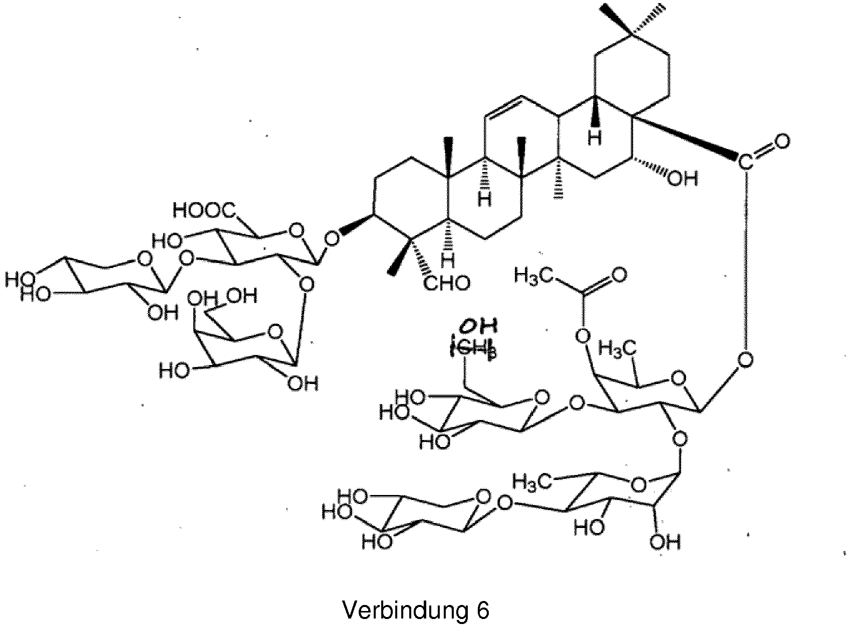

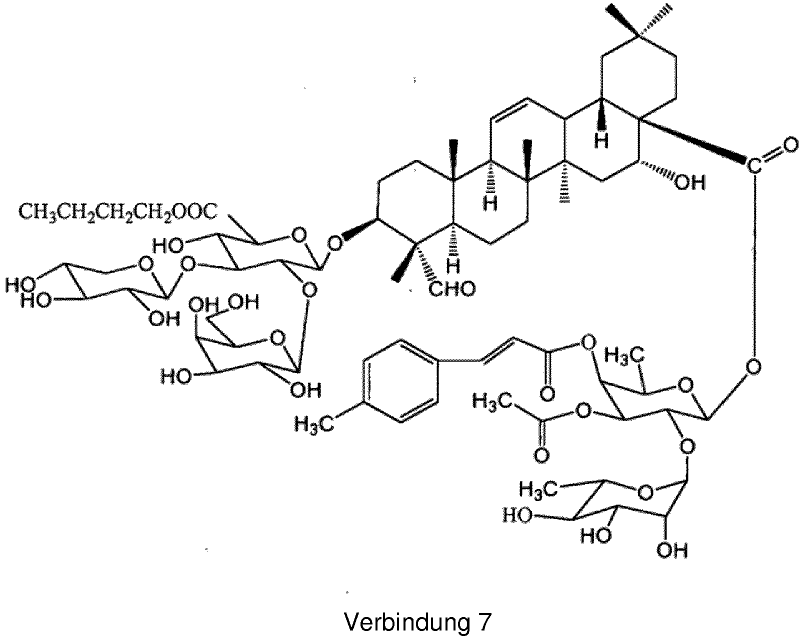

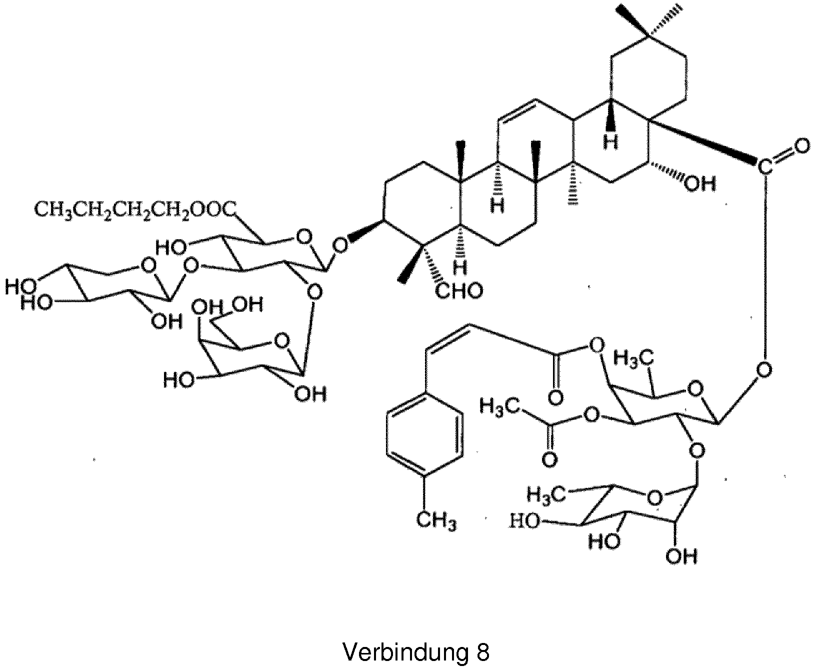

2. Verbindungen zur Verwendung nach Anspruch 1, wobei die Verbindungen allein, in einem Gemisch aus zwei oder mehreren oder gemischt mit anderen Materialien verwendet werden, um sie zu Mitteln zu verarbeiten, zum Beispiel zu Injektionen, topischen Lösungen, Salben, Pasten, Pflastern, Tropfen, Gurgelwässern, Zäpfchen, Sublingualtabletten, Pastenfilmen, Aerosolen, Brausetabletten und Pillenmitteln.

## Revendications

1. Composé de saponines de Wacao pour son utilisation dans le traitement du diabète, dans lequel ledit composé est sélectionné parmi les composés ayant la formule suivante :

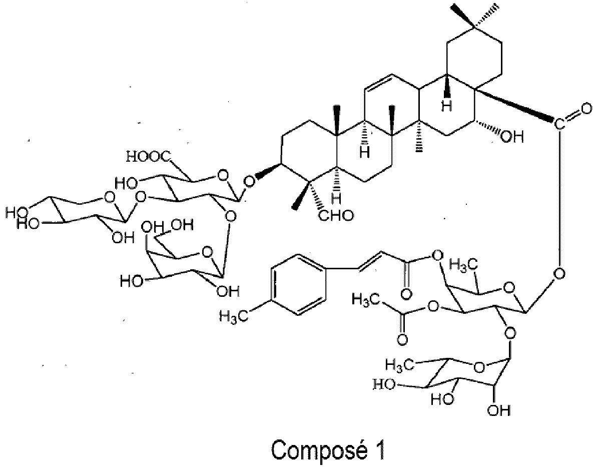

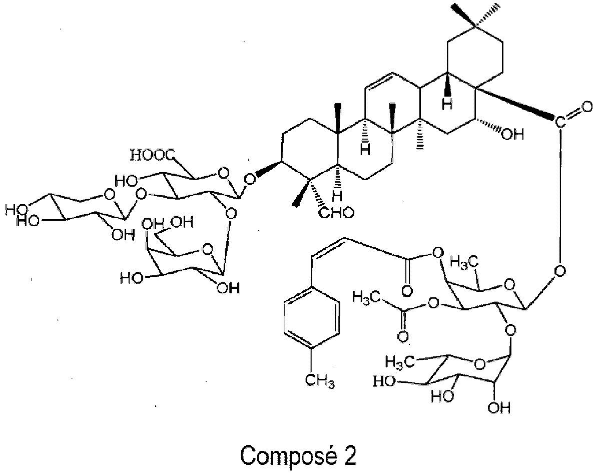

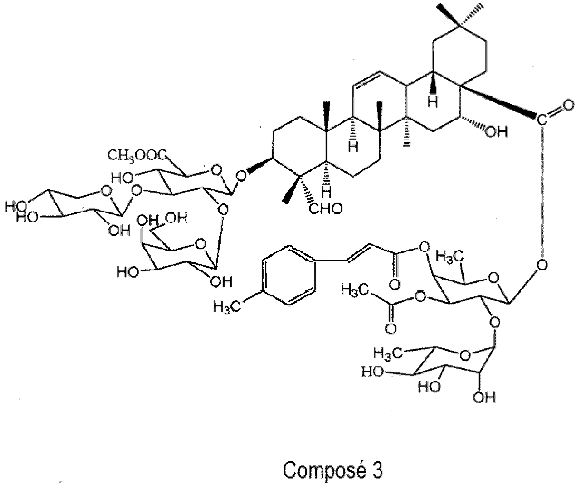

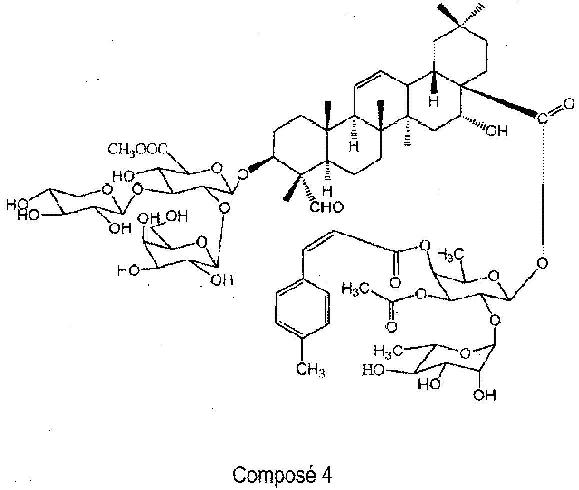

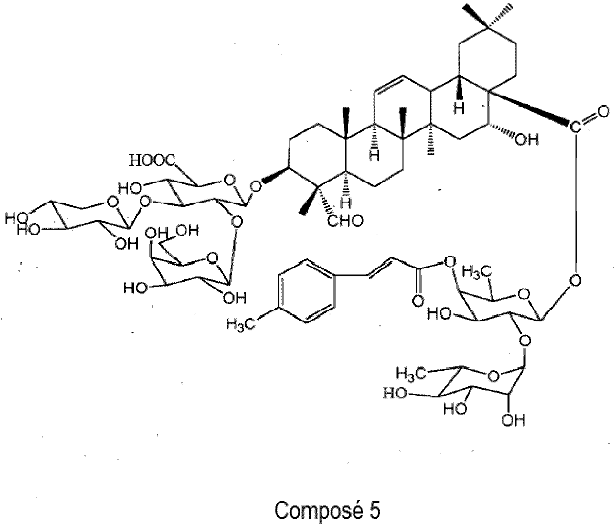

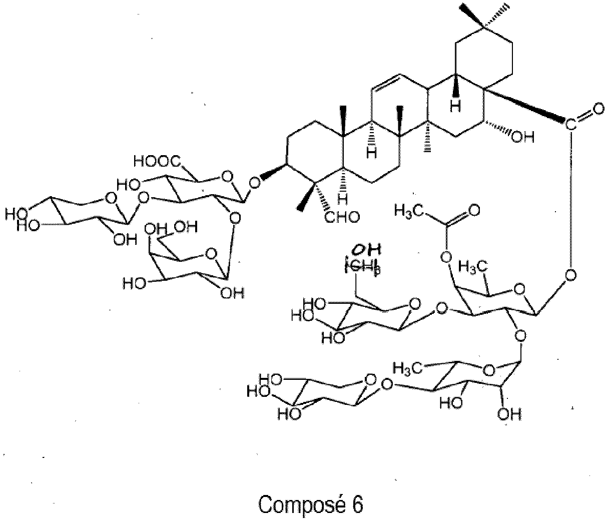

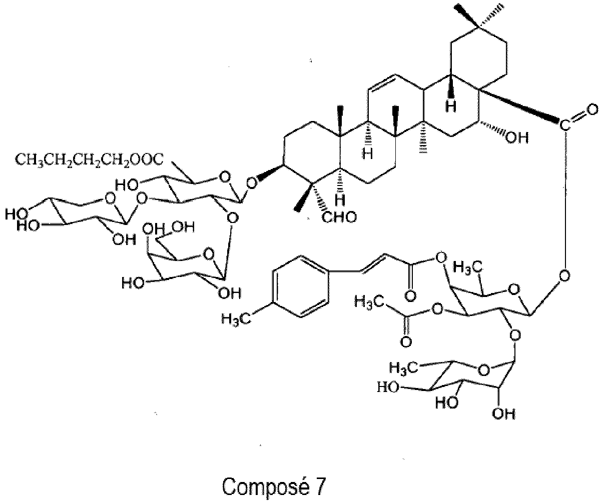

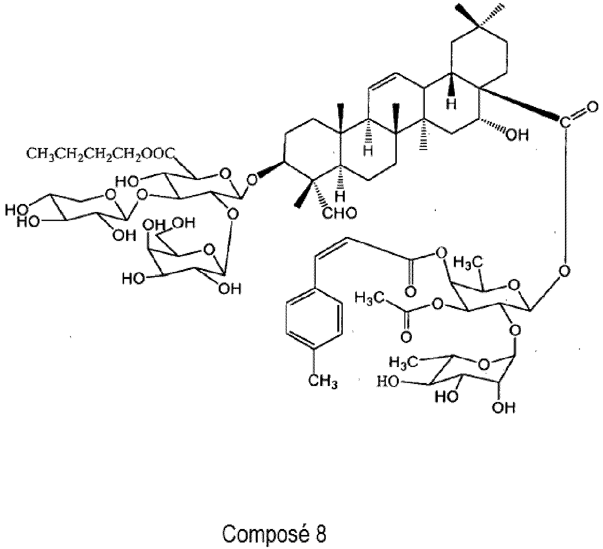

2. Composés pour leur utilisation selon la revendication 1, dans lesquels lesdits composés sont utilisés seuls, dans un mélange de deux ou plus, ou mélangés à d'autres substances pour être mis sous la forme de préparations telles que des injections, des solutions topiques, des onguents, des pâtes, des timbres, des gouttes, des gargarismes, des suppositoires, des comprimés sublinguaux, des cataplasmes, des préparations aérosol, des comprimés effervescents et des pilules.
